# EUROPEAN PATENT APPLICATION

(11) **EP 4 834 209 A2**
(43) Date of publication of application: **04.11.2026**
(21) Application number: 26206189.8
(22) Date of filing: 15.11.2021
(51) Int. Cl.: A61B 5/00

(54) **ACTIVITY CLASSIFICATION AND DISPLAY**

(30) Priority: 16.11.2020 US 202063114188 P
(62) Document number(s) of the earlier application(s) in accordance with Art. 76 EPC: 21835892.7 / 4 243 680
(71) Applicant: Oura Health Oy, 90590 Oulu (FI)
(72) Inventor: SERGEEV, Dmitry, 90590 Oulu (FI); PARTANEN, Jukka, 90590 Oulu (FI); AKHTER, Azeem, 90590 Oulu (FI); KUKKA, Matias, 90590 Oulu (FI); SINGLETON, Robert, 90590 Oulu (FI); KUKKA, Janne, 90590 Oulu (FI)
(74) Representative: J A Kemp LLP

(57) **Abstract**

Methods, systems, and devices for activity classification are described. A system may receive physiological data associated with a user via a wearable device, where the physiological data includes at least motion data. The system may identify an activity segment during which the user is engaged in a physical activity based on the motion data, where the activity segment is associated with activity segment data including at least the physiological data collected during the activity segment. The system may generate activity classification data associated with the activity segment based on the activity segment data, the activity classification data including a set of classified activity types and corresponding confidence values. The system may then cause a graphical user interface (GUI) of a user device to display the activity segment data and at least one classified activity type of the set of classified activity types.

## Description

### CROSS REFERENCE

The present Application for Patent claims the benefit of U.S. Non-Provisional Patent Application No. 17/526,300 by Sergeev et al., entitled "ACTIVITY CLASSIFICATION AND DISPLAY," filed November 15, 2021, which claims the benefit of U.S. Provisional Patent Application No. 63/114,188 by SERGEEV et al., entitled "ACTIVITY CLASSIFICATION AND DISPLAY," filed November 16, 2020, assigned to the assignee hereof, and expressly incorporated by reference herein.

### FIELD OF TECHNOLOGY

The following relates to wearable devices and data processing, including activity classification and display.

### BACKGROUND

Some wearable devices may be configured to collect physiological data from users, including temperature data, heart rate data, and the like. Many users have a desire for more insight regarding their physical health.

### BRIEF DESCRIPTION OF THE DRAWINGS

FIG. 1 illustrates an example of a system that supports activity classification and display in accordance with aspects of the present disclosure.
FIG. 2 illustrates an example of a system that supports activity classification and display in accordance with aspects of the present disclosure.
FIG. 3 illustrates an example of a process flow that supports activity classification and display in accordance with aspects of the present disclosure.
FIG. 4 illustrates an example of a process flow that supports activity classification and display in accordance with aspects of the present disclosure.
FIG. 5 illustrates an example of a system* that supports activity classification and display in accordance with aspects of the present disclosure.
FIG. 6 illustrates an example of a system that supports activity classification and display in accordance with aspects of the present disclosure.
FIG. 7 illustrates an example of a graphical user interface (GUI) that supports activity classification and display in accordance with aspects of the present disclosure.
FIG. 8 illustrates an example of a GUI that supports activity classification and display in accordance with aspects of the present disclosure.
FIG. 9 illustrates an example of a GUI that supports activity classification and display in accordance with aspects of the present disclosure.
FIG. 10 illustrates an example of an activity segment classification diagram that supports activity classification and display in accordance with aspects of the present disclosure.
FIG. 11 shows a block diagram of an apparatus that supports activity classification and display in accordance with aspects of the present disclosure.
FIG. 12 shows a block diagram of a wearable application that supports activity classification and display in accordance with aspects of the present disclosure.
FIG. 13 shows a diagram of a system including a device that supports activity classification and display in accordance with aspects of the present disclosure.
FIGs. 14 through 16 show flowcharts illustrating methods that support activity classification and display in accordance with aspects of the present disclosure.

### DETAILED DESCRIPTION

Some wearable devices may be configured to collect physiological data from users, including temperature data, motion data, and the like. Acquired physiological data may be used to analyze the user's movement and other activities, such as physical activity and exercises. Many users have a desire for more insight regarding their physical health, including their sleeping patterns, activity, and overall physical well-being. Some wearable devices may be configured to acquire data from a user, and determine when a user is engaged in physical activity. However, some conventional wearable devices may be unable to differentiate between different types of physical activity. For example, some wearable devices may collect motion data from a user which suggests that the user is engaged in some sort of physical activity, but may be unable to determine whether the user is running, swimming, on an elliptical, and the like. The inability to differentiate between different types of physical activity may lead to inaccurate activity measurements for the user, as different types of activity may exhibit varying levels of calorie consumption, physical exertion, and the like.

Accordingly, aspects of the present disclosure are directed to techniques which enable improved activity classification and display. In particular, aspects of the present disclosure are directed to a system which acquires physiological data from a user, determines when the user is engaged in a physical activity based on the acquired physiological data, and generates activity classification data for the physical activity including classified activity types and corresponding confidence values. In this regard, techniques described herein may enable the system to differentiate between different types of classified activity types (e.g., running, swimming, biking, hiking), and may assign confidence levels associated with the respective classified activity types, where the confidence values indicate a relative confidence/probability that an identified activity segment is associated with the respective classified activity type.

According to aspects of the present disclosure, a wearable device may acquire physiological data from a user, and may send acquired physiological data and otherwise communicate with a user device running an application or other software associated with the wearable device. The application may display the measured physiological data, patterns, insights, messaging, media content and the like to the user via a user interface in the application. In this regard, the wearable device may measure user physiological parameters, process the measured parameters, and provide outputs to users in a graphical user interface (GUI). For example, the wearable device may acquire a user's physiological data (e.g., motion data, temperature data, and the like) and classify a user's current activities and previous activities based on the acquired data. The activities may be an example of physical activities, such as exercises, sports, recreational activities, and physical work.

Continuing with the same example, a server associated with the wearable device may output activity classification data for a period of time during which a user is active. The activity classification data may include a plurality of activity classifications each of which includes an associated confidence level that indicates a level of confidence in the classification. For example, each activity classification may be associated with a percentage value that indicates a level of confidence (e.g., probability) that the activity classification is correct.

In some cases, the user device running the application may generate a GUI for the activity classification. The GUI may include text, images, and GUI elements (e.g., buttons, menus, etc.). The GUI associated with the activity classification may be referred to herein as an activity GUI. The GUI elements included in the activity GUI may be referred to herein as activity GUI elements. In some implementations, the activity GUI (e.g., text, images, and/or activity GUI elements) may be included as a component of a larger GUI, such as a health, wellness, and/or training GUI for an application that provides additional functionality.

The activity GUI may display information associated with the classified activities, such as activity names for recent and/or current activities, a time the activity occurred, a duration of the activity, or a combination thereof. The activity GUI elements may provide information to the user and receive user input. In some cases, the user input may be an example of a user confirmation of a classified activity and/or a user-selection of an activity from a list.

The user device running the application may render the activity GUI based on the classification data. For example, the activity GUI may include different text, images, and/or activity GUI elements based on the confidence values associated with the classifications. In some examples, the activity GUI may display the activity associated with the highest confidence value (e.g., the most likely classified activity type). In other examples, the activity GUI may include a button and/or selection GUI element (e.g., a drop-down menu) that allows the user to select and/or confirm the activity. For example, the activity GUI may provide a confirmation and/or selection GUI element where confidence levels may not be as conclusive. The text in the activity GUI may also reflect the level of confidence in the activity classification.

Aspects of the disclosure are initially described in the context of systems supporting physiological data collection from users via wearable devices. Additional aspects of the disclosure are described in the context of process flows, systems, example GUIs, and diagrams. Aspects of the disclosure are further illustrated by and described with reference to apparatus diagrams, system diagrams, and flowcharts that relate to activity classification and display.

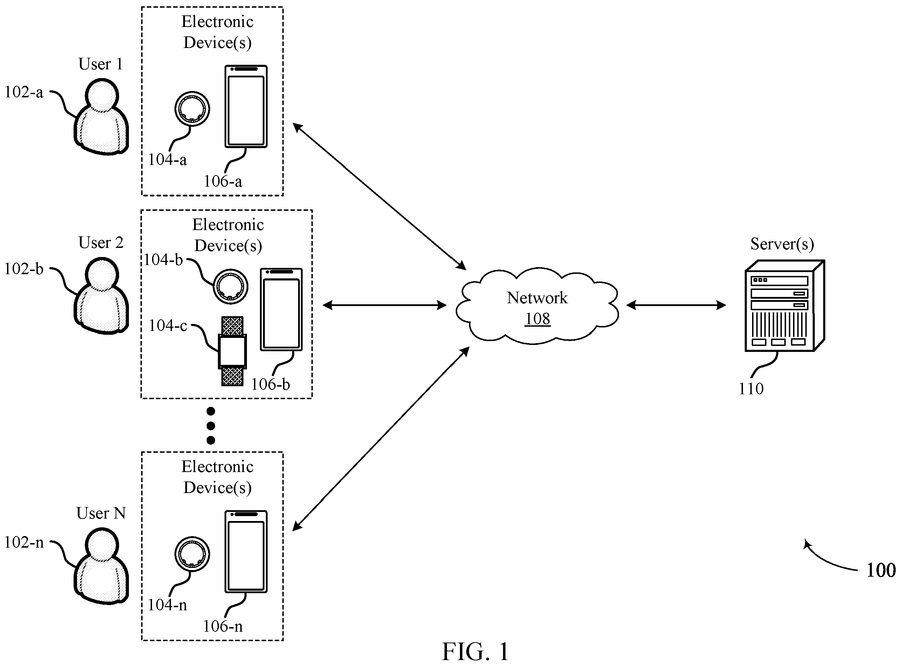

**FIG. 1** illustrates an example of a system 100 that supports activity classification and display in accordance with aspects of the present disclosure. The system 100 includes a plurality of electronic devices (e.g., wearable devices 104, user devices 106) which may be worn and/or operated by one or more users 102. The system 100 further includes a network 108 and one or more servers 110.

The electronic devices may include any electronic devices known in the art, including wearable devices 104 (e.g., ring wearable devices, watch wearable devices, etc.), user devices 106 (e.g., smartphones, laptops, tablets). The electronic devices associated with the respective users 102 may include one or more of the following functionalities: 1) measuring physiological data, 2) storing the measured data, 3) processing the data, 4) providing outputs (e.g., via GUIs) to a user 102 based on the processed data, and 5) communicating data with one another and/or other computing devices. Different electronic devices may perform one or more of the functionalities.

Example wearable devices 104 may include wearable computing devices, such as a ring computing device (hereinafter "ring") configured to be worn on a user's 102 finger, a wrist computing device (e.g., a smart watch, fitness band, or bracelet) configured to be worn on a user's 102 wrist, and/or a head mounted computing device (e.g., glasses/goggles). Wearable devices 104 may also include bands, straps (e.g., flexible or inflexible bands or straps), stick-on sensors, and the like, which may be positioned in other locations, such as bands around the head (e.g., a forehead headband), arm (e.g., a forearm band and/or bicep band), and/or leg (e.g., a thigh or calf band), behind the ear, under the armpit, and the like. Wearable devices 104 may also be attached to, or included in, articles of clothing. For example, wearable devices 104 may be included in pockets and/or pouches on clothing. As another example, wearable device 104 may be clipped and/or pinned to clothing, or may otherwise be maintained within the vicinity of the user 102. Example articles of clothing may include, but are not limited to, hats, shirts, gloves, pants, socks, outerwear (e.g., jackets), and undergarments. In some implementations, wearable devices 104 may be included with other types of devices such as training/sporting devices that are used during physical activity. For example, wearable devices 104 may be attached to, or included in, a bicycle, skis, a tennis racket, a golf club, and/or training weights.

Much of the present disclosure may be described in the context of a ring wearable device 104. Accordingly, the terms "ring 104," "wearable device 104," and like terms, may be used interchangeably, unless noted otherwise herein. However, the use of the term "ring 104" is not to be regarded as limiting, as it is contemplated herein that aspects of the present disclosure may be performed using other wearable devices (e.g., watch wearable devices, necklace wearable device, bracelet wearable devices, earring wearable devices, anklet wearable devices, and the like).

In some aspects, user devices 106 may include handheld mobile computing devices, such as smartphones and tablet computing devices. User devices 106 may also include personal computers, such as laptop and desktop computing devices. Other example user devices 106 may include server computing devices that may communicate with other electronic devices (e.g., via the Internet). In some implementations, computing devices may include medical devices, such as external wearable computing devices (e.g., Holter monitors). Medical devices may also include implantable medical devices, such as pacemakers and cardioverter defibrillators. Other example user devices 106 may include home computing devices, such as internet of things (IoT) devices (e.g., IoT devices), smart televisions, smart speakers, smart displays (e.g., video call displays), hubs (e.g., wireless communication hubs), security systems, smart appliances (e.g., thermostats and refrigerators), and fitness equipment.

Some electronic devices (e.g., wearable devices 104, user devices 106) may measure physiological parameters of respective users 102, such as photoplethysmography waveforms, continuous skin temperature, a pulse waveform, respiration rate, heart rate, heart rate variability (HRV), actigraphy, galvanic skin response, pulse oximetry, and/or other physiological parameters. Some electronic devices that measure physiological parameters may also perform some/all of the calculations described herein. Some electronic devices may not measure physiological parameters, but may perform some/all of the calculations described herein. For example, a ring (e.g., wearable device 104), mobile device application, or a server computing device may process received physiological data that was measured by other devices.

In some implementations, a user 102 may operate, or may be associated with, multiple electronic devices, some of which may measure physiological parameters and some of which may process the measured physiological parameters. In some implementations, a user 102 may have a ring (e.g., wearable device 104) that measures physiological parameters. The user 102 may also have, or be associated with, a user device 106 (e.g., mobile device, smartphone), where the wearable device 104 and the user device 106 are communicatively coupled to one another. In some cases, the user device 106 may receive data from the wearable device 104 and perform some/all of the calculations described herein. In some implementations, the user device 106 may also measure physiological parameters described herein, such as motion/activity parameters.

For example, as illustrated in FIG. 1, a first user 102-a (User 1) may operate, or may be associated with, a wearable device 104-a (e.g., ring 104-a) and a user device 106-a that may operate as described herein. In this example, the user device 106-a associated with user 102-a may process/store physiological parameters measured by the ring 104-a. Comparatively, a second user 102-b (User 2) may be associated with a ring 104-b, a watch wearable device 104-c (e.g., watch 104-c), and a user device 106-b, where the user device 106-b associated with user 102-b may process/store physiological parameters measured by the ring 104-b and/or the watch 104-c. Moreover, an nth user 102-n (User N) may be associated with an arrangement of electronic devices described herein (e.g., ring 104-n, user device 106-n). In some aspects, wearable devices 104 (e.g., rings 104, watches 104) and other electronic devices may be communicatively coupled to the user devices 106 of the respective users 102 via Bluetooth, Wi-Fi, and other wireless protocols.

In some implementations, the rings 104 (e.g., wearable devices 104) of the system 100 may be configured to collect physiological data from the respective users 102 based on arterial blood flow within the user's finger. In particular, a ring 104 may utilize one or more LEDs (e.g., red LEDs, green LEDs) which emit light on the palm-side of a user's finger to collect physiological data based on arterial blood flow within the user's finger. In some implementations, the ring 104 may acquire the physiological data using a combination of both green and red LEDs. The physiological data may include any physiological data known in the art including, but not limited to, temperature data, accelerometer data (e.g., movement/motion data), heart rate data, HRV data, blood oxygen level data, or any combination thereof.

The use of both green and red LEDs may provide several advantages over other solutions, as red and green LEDs have been found to have their own distinct advantages when acquiring physiological data under different conditions (e.g., light/dark, active/inactive) and via different parts of the body, and the like. For example, green LEDs have been found to exhibit better performance during exercise. Moreover, using multiple LEDs (e.g., green and red LEDs) distributed around the ring 104 has been found to exhibit superior performance as compared to wearable devices which utilize LEDs which are positioned close to one another, such as within a watch wearable device. Furthermore, the blood vessels in the finger (e.g., arteries, capillaries) are more accessible via LEDs as compared to blood vessels in the wrist. In particular, arteries in the wrist are positioned on the bottom of the wrist (e.g., palm-side of the wrist), meaning only capillaries are accessible on the top of the wrist (e.g., back of hand side of the wrist), where wearable watch devices and similar devices are typically worn. As such, utilizing LEDs and other sensors within a ring 104 has been found to exhibit superior performance as compared to wearable devices worn on the wrist, as the ring 104 may have greater access to arteries (as compared to capillaries), thereby resulting in stronger signals and more valuable physiological data.

The electronic devices of the system 100 (e.g., user devices 106, wearable devices 104) may be communicatively coupled to one or more servers 110 via wired or wireless communication protocols. For example, as shown in FIG. 1, the electronic devices (e.g., user devices 106) may be communicatively coupled to one or more servers 110 via a network 108. The network 108 may implement transfer control protocol and internet protocol (TCP/IP), such as the Internet, or may implement other network 108 protocols. Network connections between the network 108 and the respective electronic devices may facilitate transport of data via email, web, text messages, mail, or any other appropriate form of interaction within a computer network 108. For example, in some implementations, the ring 104-a associated with the first user 102-a may be communicatively coupled to the user device 106-a, where the user device 106-a is communicatively coupled to the servers 110 via the network 108. In additional or alternative cases, wearable devices 104 (e.g., rings 104, watches 104) may be directly communicatively coupled to the network 108.

The system 100 may offer an on-demand database service between the user devices 106 and the one or more servers 110. In some cases, the servers 110 may receive data from the user devices 106 via the network 108, and may store and analyze the data. Similarly, the servers 110 may provide data to the user devices 106 via the network 108. In some cases, the servers 110 may be located at one or more data centers. The servers 110 may be used for data storage, management, and processing. In some implementations, the servers 110 may provide a web-based interface to the user device 106 via web browsers.

In some aspects, the system 100 may detect periods of time during which a user 102 is asleep, and classify periods of time during which the user 102 is asleep into one or more sleep stages (e.g., sleep stage classification). For example, as shown in FIG. 1, User 102-a may be associated with a wearable device 104-a (e.g., ring 104-a) and a user device 106-a. In this example, the ring 104-a may collect physiological data associated with the user 102-a, including temperature, heart rate, HRV, respiratory rate, and the like. In some aspects, data collected by the ring 104-a may be input to a machine learning classifier, where the machine learning classifier is configured to determine periods of time during which the user 102-a is (or was) asleep. Moreover, the machine learning classifier may be configured to classify periods of time into different sleep stages, including an awake sleep stage, a rapid eye movement (REM) sleep stage, a light sleep stage (non-REM (NREM)), and a deep sleep stage (NREM). In some aspects, the classified sleep stages may be displayed to the user 102-a via a GUI of the user device 106-a. Sleep stage classification may be used to provide feedback to a user 102-a regarding the user's sleeping patterns, such as recommended bedtimes, recommended wake-up times, and the like. Moreover, in some implementations, sleep stage classification techniques described herein may be used to calculate scores for the respective user, such as Sleep Scores, Readiness Scores, and the like.

In some aspects, the system 100 may utilize circadian rhythm-derived features to further improve physiological data collection, data processing procedures, and other techniques described herein. The term circadian rhythm may refer to a natural, internal process that regulates an individual's sleep-wake cycle, which repeats approximately every 24 hours. In this regard, techniques described herein may utilize circadian rhythm adjustment models to improve physiological data collection, analysis, and data processing. For example, a circadian rhythm adjustment model may be input into a machine learning classifier along with physiological data collected from the user 102-a via the wearable device 104-a. In this example, the circadian rhythm adjustment model may be configured to "weight," or adjust, physiological data collected throughout a user's natural, approximately 24-hour circadian rhythm. In some implementations, the system may initially start with a "baseline" circadian rhythm adjustment model, and may modify the baseline model using physiological data collected from each user 102 to generate tailored, individualized circadian rhythm adjustment models which are specific to each respective user 102.

In some aspects, the system 100 may utilize other biological rhythms to further improve physiological data collection, analysis, and processing by phase of these other rhythms. For example, if a weekly rhythm is detected within an individual's baseline data, then the model may be configured to adjust "weights" of data by day of the week. Biological rhythms that may require adjustment to the model by this method include: 1) ultradian (faster than a day rhythms, including sleep cycles in a sleep state, and oscillations from less than an hour to several hours periodicity in the measured physiological variables during wake state; 2) circadian rhythms; 3) non-endogenous daily rhythms shown to be imposed on top of circadian rhythms, as in work schedules; 4) weekly rhythms, or other artificial time periodicities exogenously imposed (e.g. in a hypothetical culture with 12 day "weeks", 12 day rhythms could be used); 5) multi-day ovarian rhythms in women and spermatogenesis rhythms in men; 6) lunar rhythms (relevant for individuals living with low or no artificial lights); and 7) seasonal rhythms.

The biological rhythms are not always stationary rhythms. For example, many women experience variability in ovarian cycle length across cycles, and ultradian rhythms are not expected to occur at exactly the same time or periodicity across days even within a user. As such, signal processing techniques sufficient to quantify the frequency composition while preserving temporal resolution of these rhythms in physiological data may be used to improve detection of these rhythms, to assign phase of each rhythm to each moment in time measured, and to thereby modify adjustment models and comparisons of time intervals. The biological rhythm-adjustment models and parameters can be added in linear or non-linear combinations as appropriate to more accurately capture the dynamic physiological baselines of an individual or group of individuals.

In some aspects, the respective devices of the system 100 may support techniques for activity classification and display. In some cases, the respective devices of the system 100 may support aspects of the present disclosure, including techniques for acquiring a user's physiological data (e.g., motion data and/or temperature data), classifying a user's current and previous activities, and generating activity GUIs based on the classifications.

It should be appreciated by a person skilled in the art that one or more aspects of the disclosure may be implemented in a system 100 to additionally or alternatively solve other problems than those described above. Furthermore, aspects of the disclosure may provide technical improvements to "conventional" systems or processes as described herein. However, the description and appended drawings only include example technical improvements resulting from implementing aspects of the disclosure, and accordingly do not represent all of the technical improvements provided within the scope of the claims.

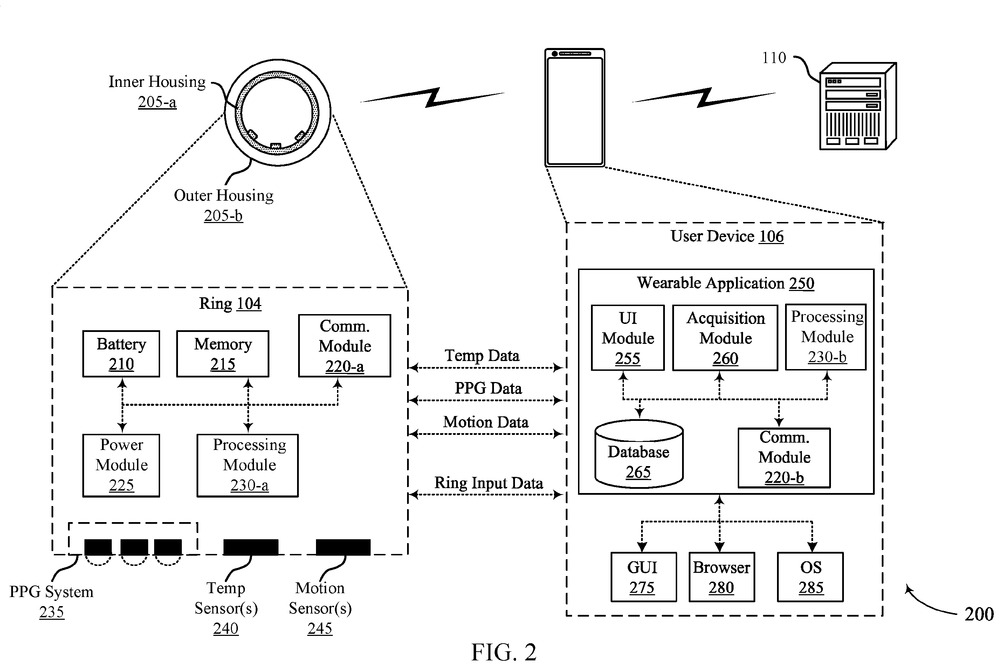

**FIG. 2** illustrates an example of a system 200 that supports activity classification and display in accordance with aspects of the present disclosure. The system 200 may implement, or be implemented by, system 100. In particular, system 200 illustrates an example of a ring 104 (e.g., wearable device 104), a user device 106, and a server 110, as described with reference to FIG. 1.

In some aspects, the ring 104 may be configured to be worn around a user's finger, and may determine one or more user physiological parameters when worn around the user's finger. Example measurements and determinations may include, but are not limited to, user skin temperature, pulse waveforms, respiratory rate, heart rate, HRV, blood oxygen levels, and the like.

System 200 further includes a user device 106 (e.g., a smartphone) in communication with the ring 104. For example, the ring 104 may be in wireless and/or wired communication with the user device 106. In some implementations, the ring 104 may send measured and processed data (e.g., temperature data, photoplethysmogram (PPG) data, motion/accelerometer data, ring input data, and the like) to the user device 106. The user device 106 may also send data to the ring 104, such as ring 104 firmware/configuration updates. The user device 106 may process data. In some implementations, the user device 106 may transmit data to the server 110 for processing and/or storage.

The ring 104 may include a housing 205, which may include an inner housing 205-a and an outer housing 205-b. In some aspects, the housing 205 of the ring 104 may store or otherwise include various components of the ring including, but not limited to, device electronics, a power source (e.g., battery 210, and/or capacitor), one or more substrates (e.g., printable circuit boards) that interconnect the device electronics and/or power source, and the like. The device electronics may include device modules (e.g., hardware/software), such as: a processing module 230-a, a memory 215, a communication module 220-a, a power module 225, and the like. The device electronics may also include one or more sensors. Example sensors may include one or more temperature sensors 240, a PPG sensor assembly (e.g., PPG system 235), and one or more motion sensors 245.

The sensors may include associated modules (not illustrated) configured to communicate with the respective components/modules of the ring 104, and generate signals associated with the respective sensors. In some aspects, each of the components/modules of the ring 104 may be communicatively coupled to one another via wired or wireless connections. Moreover, the ring 104 may include additional and/or alternative sensors or other components which are configured to collect physiological data from the user, including light sensors (e.g., LEDs), oximeters, and the like.

The ring 104 shown and described with reference to FIG. 2 is provided solely for illustrative purposes. As such, the ring 104 may include additional or alternative components as those illustrated in FIG. 2. Other rings 104 that provide functionality described herein may be fabricated. For example, rings 104 with fewer components (e.g., sensors) may be fabricated. In a specific example, a ring 104 with a single temperature sensor 240 (or other sensor), a power source, and device electronics configured to read the single temperature sensor 240 (or other sensor) may be fabricated. In another specific example, a temperature sensor 240 (or other sensor) may be attached to a user's finger (e.g., using a clamps, spring loaded clamps, etc.). In this case, the sensor may be wired to another computing device, such as a wrist worn computing device that reads the temperature sensor 240 (or other sensor). In other examples, a ring 104 that includes additional sensors and processing functionality may be fabricated.

The housing 205 may include one or more housing 205 components. The housing 205 may include an outer housing 205-b component (e.g., a shell) and an inner housing 205-a component (e.g., a molding). The housing 205 may include additional components (e.g., additional layers) not explicitly illustrated in FIG. 2. For example, in some implementations, the ring 104 may include one or more insulating layers that electrically insulate the device electronics and other conductive materials (e.g., electrical traces) from the outer housing 205-b (e.g., a metal outer housing 205-b). The housing 205 may provide structural support for the device electronics, battery 210, substrate(s), and other components. For example, the housing 205 may protect the device electronics, battery 210, and substrate(s) from mechanical forces, such as pressure and impacts. The housing 205 may also protect the device electronics, battery 210, and substrate(s) from water and/or other chemicals.

The outer housing 205-b may be fabricated from one or more materials. In some implementations, the outer housing 205-b may include a metal, such as titanium, which may provide strength and abrasion resistance at a relatively light weight. The outer housing 205-b may also be fabricated from other materials, such polymers. In some implementations, the outer housing 205-b may be protective as well as decorative.

The inner housing 205-a may be configured to interface with the user's finger. The inner housing 205-a may be formed from a polymer (e.g., a medical grade polymer) or other material. In some implementations, the inner housing 205-a may be transparent. For example, the inner housing 205-a may be transparent to light emitted by the PPG light emitting diodes (LEDs). In some implementations, the inner housing 205-a component may be molded onto the outer housing 205-a. For example, the inner housing 205-a may include a polymer that is molded (e.g., injection molded) to fit into an outer housing 205-b metallic shell.

The ring 104 may include one or more substrates (not illustrated). The device electronics and battery 210 may be included on the one or more substrates. For example, the device electronics and battery 210 may be mounted on one or more substrates. Example substrates may include one or more printed circuit boards (PCBs), such as flexible PCB (e.g., polyimide). In some implementations, the electronics/battery 210 may include surface mounted devices (e.g., surface-mount technology (SMT) devices) on a flexible PCB. In some implementations, the one or more substrates (e.g., one or more flexible PCBs) may include electrical traces that provide electrical communication between device electronics. The electrical traces may also connect the battery 210 to the device electronics.

The device electronics, battery 210, and substrates may be arranged in the ring 104 in a variety of ways. In some implementations, one substrate that includes device electronics may be mounted along the bottom of the ring 104 (e.g., the bottom half), such that the sensors (e.g., PPG system 235, temperature sensors 240, motion sensors 245, and other sensors) interface with the underside of the user's finger. In these implementations, the battery 210 may be included along the top portion of the ring 104 (e.g., on another substrate).

The various components/modules of the ring 104 represent functionality (e.g., circuits and other components) that may be included in the ring 104. Modules may include any discrete and/or integrated electronic circuit components that implement analog and/or digital circuits capable of producing the functions attributed to the modules herein. For example, the modules may include analog circuits (e.g., amplification circuits, filtering circuits, analog/digital conversion circuits, and/or other signal conditioning circuits). The modules may also include digital circuits (e.g., combinational or sequential logic circuits, memory circuits etc.).

The memory 215 (memory module) of the ring 104 may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. The memory 215 may store any of the data described herein. For example, the memory 215 may be configured to store data (e.g., motion data, temperature data, PPG data) collected by the respective sensors and PPG system 235. Furthermore, memory 215 may include instructions that, when executed by one or more processing circuits, cause the modules to perform various functions attributed to the modules herein. The device electronics of the ring 104 described herein are only example device electronics. As such, the types of electronic components used to implement the device electronics may vary based on design considerations.

The functions attributed to the modules of the ring 104 described herein may be embodied as one or more processors, hardware, firmware, software, or any combination thereof. Depiction of different features as modules is intended to highlight different functional aspects and does not necessarily imply that such modules must be realized by separate hardware/software components. Rather, functionality associated with one or more modules may be performed by separate hardware/software components or integrated within common hardware/software components.

The processing module 230-a of the ring 104 may include one or more processors (e.g., processing units), microcontrollers, digital signal processors, systems on a chip (SOCs), and/or other processing devices. The processing module 230-a communicates with the modules included in the ring 104. For example, the processing module 230-a may transmit/receive data to/from the modules and other components of the ring 104, such as the sensors. As described herein, the modules may be implemented by various circuit components. Accordingly, the modules may also be referred to as circuits (e.g., a communication circuit and power circuit).

The processing module 230-a may communicate with the memory 215. The memory 215 may include computer-readable instructions that, when executed by the processing module 230-a, cause the processing module 230-a to perform the various functions attributed to the processing module 230-a herein. In some implementations, the processing module 230-a (e.g., a microcontroller) may include additional features associated with other modules, such as communication functionality provided by the communication module 220-a (e.g., an integrated Bluetooth Low Energy transceiver) and/or additional onboard memory 215.

The communication module 220-a may include circuits that provide wireless and/or wired communication with the user device 106 (e.g., communication module 220-b of the user device 106). In some implementations, the communication modules 220-a, 220-b may include wireless communication circuits, such as Bluetooth circuits and/or Wi-Fi circuits. In some implementations, the communication modules 220-a, 220-b can include wired communication circuits, such as Universal Serial Bus (USB) communication circuits. Using the communication module 220-a, the ring 104 and the user device 106 may be configured to communicate with each other. The processing module 230-a of the ring may be configured to transmit/receive data to/from the user device 106 via the communication module 220-a. Example data may include, but is not limited to, motion data, temperature data, pulse waveforms, heart rate data, HRV data, PPG data, and status updates (e.g., charging status, battery charge level, and/or ring 104 configuration settings). The processing module 230-a of the ring may also be configured to receive updates (e.g., software/firmware updates) and data from the user device 106.

The ring 104 may include a battery 210 (e.g., a rechargeable battery 210). An example battery 210 may include a Lithium-Ion or Lithium-Polymer type battery 210, although a variety of battery 210 options are possible. The battery 210 may be wirelessly charged. In some implementations, the ring 104 may include a power source other than the battery 210, such as a capacitor. The power source (e.g., battery 210 or capacitor) may have a curved geometry that matches the curve of the ring 104. In some aspects, a charger or other power source may include additional sensors which may be used to collect data in addition to, or which supplements, data collected by the ring 104 itself. Moreover, a charger or other power source for the ring 104 may function as a user device 106, in which case the charger or other power source for the ring 104 may be configured to receive data from the ring 104, store and/or process data received from the ring 104, and communicate data between the ring 104 and the servers 110.

In some aspects, the ring 104 includes a power module 225 that may control charging of the battery 210. For example, the power module 225 may interface with an external wireless charger that charges the battery 210 when interfaced with the r**i**ng 104. The charger may include a datum structure that mates with a ring 104 datum structure to create a specified orientation with the ring 104 during 104 charging. The power module 225 may also regulate voltage(s) of the device electronics, regulate power output to the device electronics, and monitor the state of charge of the battery 210. In some implementations, the battery 210 may include a protection circuit module (PCM) that protects the battery 210 from high current discharge, over voltage during 104 charging, and under voltage during 104 discharge. The power module 225 may also include electro-static discharge (ESD) protection.

The one or more temperature sensors 240 may be electrically coupled to the processing module 230-a. The temperature sensor 240 may be configured to generate a temperature signal (e.g., temperature data) that indicates a temperature read or sensed by the temperature sensor 240. The processing module 230-a may determine a temperature of the user in the location of the temperature sensor 240. For example, in the ring 104, temperature data generated by the temperature sensor 240 may indicate a temperature of a user at the user's finger (e.g., skin temperature). In some implementations, the temperature sensor 240 may contact the user's skin. In other implementations, a portion of the housing 205 (e.g., the inner housing 205-a) may form a barrier (e.g., a thin, thermally conductive barrier) between the temperature sensor 240 and the user's skin. In some implementations, portions of the ring 104 configured to contact the user's finger may have thermally conductive portions and thermally insulative portions. The thermally conductive portions may conduct heat from the user's finger to the temperature sensors 240. The thermally insulative portions may insulate portions of the ring 104 (e.g., the temperature sensor 240) from ambient temperature.

In some implementations, the temperature sensor 240 may generate a digital signal (e.g., temperature data) that the processing module 230-a may use to determine the temperature. As another example, in cases where the temperature sensor 240 includes a passive sensor, the processing module 230-a (or a temperature sensor 240 module) may measure a current/voltage generated by the temperature sensor 240 and determine the temperature based on the measured current/voltage. Example temperature sensors 240 may include a thermistor, such as a negative temperature coefficient (NTC) thermistor, or other types of sensors including resistors, transistors, diodes, and/or other electrical/electronic components.

The processing module 230-a may sample the user's temperature over time. For example, the processing module 230-a may sample the user's temperature according to a sampling rate. An example sampling rate may include one sample per second, although the processing module 230-a may be configured to sample the temperature signal at other sampling rates that are higher or lower than one sample per second. In some implementations, the processing module 230-a may sample the user's temperature continuously throughout the day and night. Sampling at a sufficient rate (e.g., one sample per second) throughout the day may provide sufficient temperature data for analysis described herein.

The processing module 230-a may store the sampled temperature data in memory 215. In some implementations, the processing module 230-a may process the sampled temperature data. For example, the processing module 230-a may determine average temperature values over a period of time. In one example, the processing module 230-a may determine an average temperature value each minute by summing all temperature values collected over the minute and dividing by the number of samples over the minute. In a specific example where the temperature is sampled at one sample per second, the average temperature may be a sum of all sampled temperatures for one minute divided by sixty seconds. The memory 215 may store the average temperature values over time. In some implementations, the memory 215 may store average temperatures (e.g., one per minute) instead of sampled temperatures in order to conserve memory 215.

The sampling rate, which may be stored in memory 215, may be configurable. In some implementations, the sampling rate may be the same throughout the day and night. In other implementations, the sampling rate may be changed throughout the day/night. In some implementations, the ring 104 may filter/reject temperature readings, such as large spikes in temperature that are not indicative of physiological changes (e.g., a temperature spike from a hot shower). In some implementations, the ring 104 may filter/reject temperature readings that may not be reliable due to other factors, such as excessive motion during 104 exercise (e.g., as indicated by a motion sensor 245).

The ring 104 (e.g., communication module) may transmit the sampled and/or average temperature data to the user device 106 for storage and/or further processing. The user device 106 may transfer the sampled and/or average temperature data to the server 110 for storage and/or further processing.

Although the ring 104 is illustrated as including a single temperature sensor 240, the ring 104 may include multiple temperature sensors 240 in one or more locations, such as arranged along the inner housing 205-a near the user's finger. In some implementations, the temperature sensors 240 may be stand-alone temperature sensors 240. Additionally, or alternatively, one or more temperature sensors 240 may be included with other components (e.g., packaged with other components), such as with the accelerometer and/or processor.

The processing module 230-a may acquire and process data from multiple temperature sensors 240 in a similar manner described with respect to a single temperature sensor 240. For example, the processing module 230 may individually sample, average, and store temperature data from each of the multiple temperature sensors 240. In other examples, the processing module 230-a may sample the sensors at different rates and average/store different values for the different sensors. In some implementations, the processing module 230-a may be configured to determine a single temperature based on the average of two or more temperatures determined by two or more temperature sensors 240 in different locations on the finger.

The temperature sensors 240 on the ring 104 may acquire distal temperatures at the user's finger (e.g., any finger). For example, one or more temperature sensors 240 on the ring 104 may acquire a user's temperature from the underside of a finger or at a different location on the finger. In some implementations, the ring 104 may continuously acquire distal temperature (e.g., at a sampling rate). Although distal temperature measured by a ring 104 at the finger is described herein, other devices may measure temperature at the same/different locations. In some cases, the distal temperature measured at a user's finger may differ from the temperature measured at a user's wrist or other external body location. Additionally, the distal temperature measured at a user's finger (e.g., a "shell" temperature) may differ from the user's core temperature. As such, the ring 104 may provide a useful temperature signal that may not be acquired at other internal/external locations of the body. In some cases, continuous temperature measurement at the finger may capture temperature fluctuations (e.g., small or large fluctuations) that may not be evident in core temperature. For example, continuous temperature measurement at the finger may capture minute-to-minute or hour-to-hour temperature fluctuations that provide additional insight that may not be provided by other temperature measurements elsewhere in the body.

The ring 104 may include a PPG system 235. The PPG system 235 may include one or more optical transmitters that transmit light. The PPG system 235 may also include one or more optical receivers that receive light transmitted by the one or more optical transmitters. An optical receiver may generate a signal (hereinafter "PPG" signal) that indicates an amount of light received by the optical receiver. The optical transmitters may illuminate a region of the user's finger. The PPG signal generated by the PPG system 235 may indicate the perfusion of blood in the illuminated region. For example, the PPG signal may indicate blood volume changes in the illuminated region caused by a user's pulse pressure. The processing module 230-a may sample the PPG signal and determine a user's pulse waveform based on the PPG signal. The processing module 230-a may determine a variety of physiological parameters based on the user's pulse waveform, such as a user's respiratory rate, heart rate, HRV, oxygen saturation, and other circulatory parameters.

In some implementations, the PPG system 235 may be configured as a reflective PPG system 235 in which the optical receiver(s) receive transmitted light that is reflected through the region of the user's finger. In some implementations, the PPG system 235 may be configured as a transmissive PPG system 235 in which the optical transmitter(s) and optical receiver(s) are arranged opposite to one another, such that light is transmitted directly through a portion of the user's finger to the optical receiver(s).

The number and ratio of transmitters and receivers included in the PPG system 235 may vary. Example optical transmitters may include light-emitting diodes (LEDs). The optical transmitters may transmit light in the infrared spectrum and/or other spectrums. Example optical receivers may include, but are not limited to, photosensors, phototransistors, and photodiodes. The optical receivers may be configured to generate PPG signals in response to the wavelengths received from the optical transmitters. The location of the transmitters and receivers may vary. Additionally, a single device may include reflective and/or transmissive PPG systems 235.

The PPG system 235 illustrated in FIG. 2 may include a reflective PPG system 235 in some implementations. In these implementations, the PPG system 235 may include a centrally located optical receiver (e.g., at the bottom of the ring 104) and two optical transmitters located on each side of the optical receiver. In this implementation, the PPG system 235 (e.g., optical receiver) may generate the PPG signal based on light received from one or both of the optical transmitters. In other implementations, other placements, combinations, and/or configurations of one or more optical transmitters and/or optical receivers are contemplated.

The processing module 230-a may control one or both of the optical transmitters to transmit light while sampling the PPG signal generated by the optical receiver. In some implementations, the processing module 230-a may cause the optical transmitter with the stronger received signal to transmit light while sampling the PPG signal generated by the optical receiver. For example, the selected optical transmitter may continuously emit light while the PPG signal is sampled at a sampling rate (e.g., 250 Hz).

Sampling the PPG signal generated by the PPG system 235 may result in a pulse waveform, which may be referred to as a "PPG." The pulse waveform may indicate blood pressure vs time for multiple cardiac cycles. The pulse waveform may include peaks that indicate cardiac cycles. Additionally, the pulse waveform may include respiratory induced variations that may be used to determine respiration rate. The processing module 230-a may store the pulse waveform in memory 215 in some implementations. The processing module 230-a may process the pulse waveform as it is generated and/or from memory 215 to determine user physiological parameters described herein.

The processing module 230-a may determine the user's heart rate based on the pulse waveform. For example, the processing module 230-a may determine heart rate (e.g., in beats per minute) based on the time between peaks in the pulse waveform. The time between peaks may be referred to as an interbeat interval (IBI). The processing module 230-a may store the determined heart rate values and IBI values in memory 215.

The processing module 230-a may determine HRV over time. For example, the processing module 230-a may determine HRV based on the variation in the IBls. The processing module 230-a may store the HRV values over time in the memory 215. Moreover, the processing module 230-a may determine the user's respiratory rate over time. For example, the processing module 230-a may determine respiratory rate based on frequency modulation, amplitude modulation, or baseline modulation of the user's IBI values over a period of time. Respiratory rate may be calculated in breaths per minute or as another breathing rate (e.g., breaths per 30 seconds). The processing module 230-a may store user respiratory rate values over time in the memory 215.

The ring 104 may include one or more motion sensors 245, such as one or more accelerometers (e.g., 6-D accelerometers) and/or one or more gyroscopes (gyros). The motion sensors 245 may generate motion signals that indicate motion of the sensors. For example, the ring 104 may include one or more accelerometers that generate acceleration signals that indicate acceleration of the accelerometers. As another example, the ring 104 may include one or more gyro sensors that generate gyro signals that indicate angular motion (e.g., angular velocity) and/or changes in orientation. The motion sensors 245 may be included in one or more sensor packages. An example accelerometer/gyro sensor is a Bosch BMl160 inertial micro electro-mechanical system (MEMS) sensor that may measure angular rates and accelerations in three perpendicular axes.

The processing module 230-a may sample the motion signals at a sampling rate (e.g., 50Hz) and determine the motion of the ring 104 based on the sampled motion signals. For example, the processing module 230-a may sample acceleration signals to determine acceleration of the ring 104. As another example, the processing module 230-a may sample a gyro signal to determine angular motion. In some implementations, the processing module 230-a may store motion data in memory 215. Motion data may include sampled motion data as well as motion data that is calculated based on the sampled motion signals (e.g., acceleration and angular values).

The ring 104 may store a variety of data described herein. For example, the ring 104 may store temperature data, such as raw sampled temperature data and calculated temperature data (e.g., average temperatures). As another example, the ring 104 may store PPG signal data, such as pulse waveforms and data calculated based on the pulse waveforms (e.g., heart rate values, IBI values, HRV values, and respiratory rate values). The ring 104 may also store motion data, such as sampled motion data that indicates linear and angular motion.

The ring 104, or other computing device, may calculate and store additional values based on the sampled/calculated physiological data. For example, the processing module 230 may calculate and store various metrics, such as sleep metrics (e.g., a Sleep Score), activity metrics, and readiness metrics. In some implementations, additional values/metrics may be referred to as "derived values." The ring 104, or other computing/wearable device, may calculate a variety of values/metrics with respect to motion. Example derived values for motion data may include, but are not limited to, motion count values, regularity values, intensity values, metabolic equivalence of task values (METs), and orientation values. Motion counts, regularity values, intensity values, and METs may indicate an amount of user motion (e.g., velocity/acceleration) over time. Orientation values may indicate how the ring 104 is oriented on the user's finger and if the ring 104 is worn on the left hand or right hand.

In some implementations, motion counts and regularity values may be determined by counting a number of acceleration peaks within one or more periods of time (e.g., one or more 30 second to 1 minute periods). Intensity values may indicate a number of movements and the associated intensity (e.g., acceleration values) of the movements. The intensity values may be categorized as low, medium, and high, depending on associated threshold acceleration values. METs may be determined based on the intensity of movements during a period of time (e.g., 30 seconds), the regularity/irregularity of the movements, and the number of movements associated with the different intensities.

In some implementations, the processing module 230-a may compress the data stored in memory 215. For example, the processing module 230-a may delete sampled data after making calculations based on the sampled data. As another example, the processing module 230-a may average data over longer periods of time in order to reduce the number of stored values. In a specific example, if average temperatures for a user over one minute are stored in memory 215, the processing module 230-a may calculate average temperatures over a five minute time period for storage, and then subsequently erase the one minute average temperature data. The processing module 230-a may compress data based on a variety of factors, such as the total amount of used/available memory 215 and/or an elapsed time since the ring 104 last transmitted the data to the user device 106.

Although a user's physiological parameters may be measured by sensors included on a ring 104, other devices may measure a user's physiological parameters. For example, although a user's temperature may be measured by a temperature sensor 240 included in a ring 104, other devices may measure a user's temperature. In some examples, other wearable devices (e.g., wrist devices) may include sensors that measure user physiological parameters. Additionally, medical devices, such as external medical devices (e.g., wearable medical devices) and/or implantable medical devices, may measure a user's physiological parameters. One or more sensors on any type of computing device may be used to implement the techniques described herein.

The physiological measurements may be taken continuously throughout the day and/or night. In some implementations, the physiological measurements may be taken during 104 portions of the day and/or portions of the night. In some implementations, the physiological measurements may be taken in response to determining that the user is in a specific state, such as an active state, resting state, and/or a sleeping state. For example, the ring 104 can make physiological measurements in a resting/sleep state in order to acquire cleaner physiological signals. In one example, the ring 104 or other device/system may detect when a user is resting and/or sleeping and acquire physiological parameters (e.g., temperature) for that detected state. The devices/systems may use the resting/sleep physiological data and/or other data when the user is in other states in order to implement the techniques of the present disclosure.

In some implementations, as described previously herein, the ring 104 may be configured to collect, store, and/or process data, and may transfer any of the data described herein to the user device 106 for storage and/or processing. In some aspects, the user device 106 includes a wearable application 250, an operating system (OS), a web browser application (e.g., web browser 280), one or more additional applications, and a GUI 275. The user device 106 may further include other modules and components, including sensors, audio devices, haptic feedback devices, and the like. The wearable application 250 may include an example of an application (e.g., "app") which may be installed on the user device 106. The wearable application 250 may be configured to acquire data from the ring 104, store the acquired data, and process the acquired data as described herein. For example, the wearable application 250 may include a user interface (UI) module 255, an acquisition module 260, a processing module 230-b, a communication module 220-b, and a storage module (e.g., database 265) configured to store application data.

The various data processing operations described herein may be performed by the ring 104, the user device 106, the servers 110, or any combination thereof. For example, in some cases, data collected by the ring 104 may be pre-processed and transmitted to the user device 106. In this example, the user device 106 may perform some data processing operations on the received data, may transmit the data to the servers 110 for data processing, or both. For instance, in some cases, the user device 106 may perform processing operations which require relatively low processing power and/or operations which require a relatively low latency, whereas the user device 106 may transmit the data to the servers 110 for processing operations which require relatively high processing power and/or operations which may allow relatively higher latency.

In some aspects, the ring 104, user device 106, and server 110 of the system 200 may be configured to evaluate sleep patterns for a user. In particular, the respective components of the system 200 may be used to collect data from a user via the ring 104, and generate one or more scores (e.g., Sleep Score, Readiness Score) for the user based on the collected data. For example, as noted previously herein, the ring 104 of the system 200 may be worn by a user to collect data from the user, including temperature, heart rate, HRV, and the like. Data collected by the ring 104 may be used to determine when the user is asleep in order to evaluate the user's sleep for a given "sleep day." In some aspects, scores may be calculated for the user for each respective sleep day, such that a first sleep day is associated with a first set of scores, and a second sleep day is associated with a second set of scores. Scores may be calculated for each respective sleep day based on data collected by the ring 104 during the respective sleep day. Scores may include, but are not limited to, Sleep Scores, Readiness Scores, and the like.

In some cases, "sleep days" may align with the traditional calendar days, such that a given sleep day runs from midnight to midnight of the respective calendar day. In other cases, sleep days may be offset relative to calendar days. For example, sleep days may run from 6:00 pm (18:00) of a calendar day until 6:00 pm (18:00) of the subsequent calendar day. In this example, 6:00 pm may serve as a "cut-off time," where data collected from the user before 6:00 pm is counted for the current sleep day, and data collected from the user after 6:00 pm is counted for the subsequent sleep day. Due to the fact that most individuals sleep the most at night, offsetting sleep days relative to calendar days may enable the system 200 to evaluate sleep patterns for users in such a manner which is consistent with their sleep schedules. In some cases, users may be able to selectively adjust (e.g., via the GUI) a timing of sleep days relative to calendar days so that the sleep days are aligned with the duration of time in which the respective users typically sleep.

In some implementations, each overall score for a user for each respective day (e.g., Sleep Score, Readiness Score) may be determined/calculated based on one or more "contributors," "factors," or "contributing factors." For example, a user's overall Sleep Score may be calculated based on a set of contributors, including: total sleep, efficiency, restfulness, REM sleep, deep sleep, latency, timing, or any combination thereof. The Sleep Score may include any quantity of contributors. The "total sleep" contributor may refer to the sum of all sleep periods of the sleep day. The "efficiency" contributor may reflect the percentage of time spent asleep compared to time spent awake while in bed, and may be calculated using the efficiency average of long sleep periods (e.g., primary sleep period) of the sleep day, weighted by a duration of each sleep period. The "restfulness" contributor may indicate how restful the user's sleep is, and may be calculated using the average of all sleep periods of the sleep day, weighted by a duration of each period. The restfulness contributor may be based on a "wake up count" (e.g., sum of all the wake-ups (when user wakes up) detected during different sleep periods), excessive movement, and a "got up count" (e.g., sum of all the got-ups (when user gets out of bed) detected during the different sleep periods).

The "REM sleep" contributor may refer to a sum total of REM sleep durations across all sleep periods of the sleep day including REM sleep. Similarly, the "deep sleep" contributor may refer to a sum total of deep sleep durations across all sleep periods of the sleep day including deep sleep. The "latency" contributor may signify how long (e.g., average, median, longest) the user takes to go to sleep, and may be calculated using the average of long sleep periods throughout the sleep day, weighted by a duration of each period and the number of such periods (e.g., consolidation of a given sleep stage or sleep stages may be its own contributor or weight other contributors). Lastly, the "timing" contributor may refer to a relative timing of sleep periods within the sleep day and/or calendar day, and may be calculated using the average of all sleep periods of the sleep day, weighted by a duration of each period.

By way of another example, a user's overall Readiness Score may be calculated based on a set of contributors, including: sleep, sleep balance, heart rate, HRV balance, recovery index, temperature, activity, activity balance, or any combination thereof. The Readiness Score may include any quantity of contributors. The "sleep" contributor may refer to the combined Sleep Score of all sleep periods within the sleep day. The "sleep balance" contributor may refer to a cumulative duration of all sleep periods within the sleep day. In particular, sleep balance may indicate to a user whether the sleep that the user has been getting over some duration of time (e.g., the past two weeks) is in balance with the user's needs. Typically, adults need 7-9 hours of sleep a night to stay healthy, alert, and to perform at their best both mentally and physically. However, it is normal to have an occasional night of bad sleep, so the sleep balance contributor takes into account long-term sleep patterns to determine whether each user's sleep needs are being met. The "resting heart rate" contributor may indicate a lowest heart rate from the longest sleep period of the sleep day (e.g., primary sleep period) and/or the lowest heart rate from naps occurring after the primary sleep period.

Continuing with reference to the "contributors" (e.g., factors, contributing factors) of the Readiness Score, the "HRV balance" contributor may indicate a highest HRV average from the primary sleep period and the naps happening after the primary sleep period. The HRV balance contributor may help users keep track of their recovery status by comparing their HRV trend over a first time period (e.g., two weeks) to an average HRV over some second, longer time period (e.g., three months). The "recovery index" contributor may be calculated based on the longest sleep period. Recovery index measures how long it takes for a user's resting heart rate to stabilize during the night. A sign of a very good recovery is that the user's resting heart rate stabilizes during the first half of the night, at least six hours before the user wakes up, leaving the body time to recover for the next day. The "body temperature" contributor may be calculated based on the longest sleep period (e.g., primary sleep period) or based on a nap happening after the longest sleep period if the user's highest temperature during the nap is at least 0.5°C higher than the highest temperature during the longest period. In some aspects, the ring may measure a user's body temperature while the user is asleep, and the system 200 may display the user's average temperature relative to the user's baseline temperature. If a user's body temperature is outside of their normal range (e.g., clearly above or below 0.0), the body temperature contributor may be highlighted (e.g., go to a "Pay attention" state) or otherwise generate an alert for the user.

In some aspects, the system 200 may support techniques for activity classification and display. In some examples, the wearable device 104 may acquire user physiological data and send the data to a user device 106 (e.g., a smartphone). The user device 106 may provide data to a server 110 (e.g., via a wireless network) that classifies one or more act**i**vities based on the physiological data. Activity classification data generated by the servers 110 may include one or more classified activity types and corresponding confidence values associated with each respective classified activity type. In this regard, the server 110 may what type of physical activity the user is or was engaged in, and may assign confidence values to each classified activity type which indicate a relative likelihood or probability that the respective classified activity type is correct. In such cases, the user device 106 may generate an activity GUI based on the classification data received from the server 110, where the activity GUI displays classified activity types, corresponding confidence values, or both.

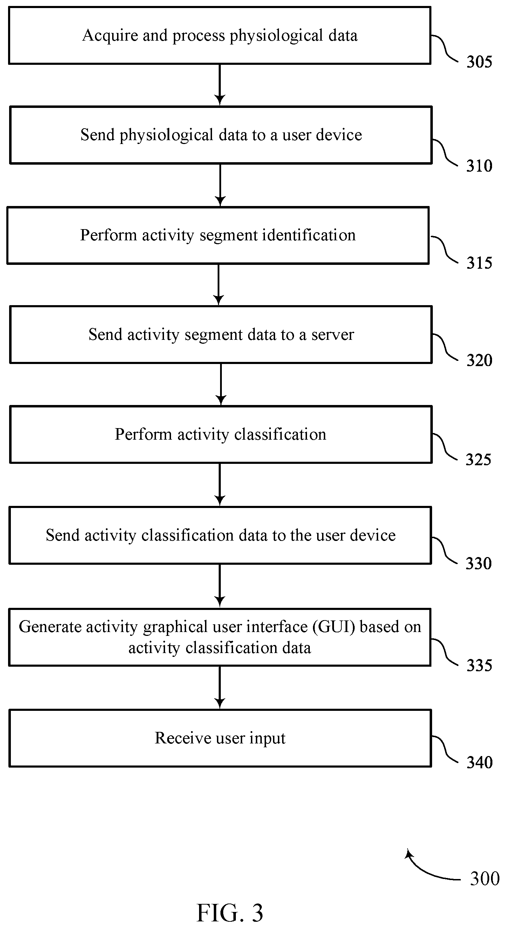

**FIG. 3** illustrates an example of process flow 300 that supports activity classification and display in accordance with aspects of the present disclosure. The process flow 300 may be implemented by the system 200 including at least a server 110, a user device 106, a wearable device 104, or some combination of components from these devices. Alternative examples of the following may be implemented, where some steps are performed in a different order than described or not performed at all. In some cases, steps may include additional features not mentioned below, or further steps may be added. Process flow 300 may describe activity classification operations and generation of activity GUIs based on the classifications.

At 305, the system 200 (e.g., wearable device 104) may acquire and process physiological data. In some implementations, the system 200 may process raw motion data and raw temperature data. For example, the system 200 may generate average values of the motion data and average values of the temperature data over a period of time. The system 200 may determine an average temperature over a period of time (e.g., each 30 second interval or 1 minute interval). In some cases, the system 200 may determine an average acceleration value and/or gyro value over a period of time (e.g., each 30 second interval or 1 minute interval).

At 310, the system 200 (e.g., wearable device 104) may send the physiological data to a user device 106 via a wireless connection. For example, the user device 106 may receive physiological data associated with the user via the wearable device 104. The physiological data may include at least motion data and temperature data. The transfer of data between the wearable device 104 and the user device 106 may be referred to as a synchronization or synch between the wearable device 104 and the user device 106. In some implementations, the wearable device 104 may send data to the user device 106 as the wearable device 104 generates the data.

The data acquired by the user device 106 may be a time series of motion data, temperature data, and/or other physiological data. The amount of data (e.g., a length of time and/or number of data points) acquired by the user device 106 may depend on how often the user device 106 acquires the motion data, temperature data, and other data. In some cases, the data may be acquired over a relatively short period of time. For example, the data may be acquired as the data is generated by the wearable device 104. In some examples, the data may be acquired over a duration of minutes, hours, a single day, or multiple days. The user device 106 may store the data as the data is acquired. As such, the user device 106 may store a time series of data (e.g., hours, days, weeks, or longer) that includes data received from the wearable device 104 in multiple segments over time.

For example, the wearable device 104 may send data to the user device 106 at predetermined intervals. In such cases, the wearable device 104 may send data to the user device 106 when the processed motion data and/or temperature data are available. In some examples, the processed temperature data and/or motion data may be available every 30 seconds. In such cases, the wearable device 104 may send the processed temperature data and/or motion data to the user device 106 every 30 seconds. In some implementations, the wearable device 104 may send the motion data and the temperature data at the same time. In other implementations, the wearable device 104 may generate and send motion data and temperature data at different time intervals when the motion data and temperature data are acquired by the wearable device 104 at different intervals.

In some implementations, the user device 106 may be configured to request data from the wearable device 104. For example, the user device 106 may be configured to request data from the wearable device 104 upon opening of the application (e.g., wearable application 250). In some examples, the user device 106 may be configured to request data from the wearable device 104 at predetermined intervals. In some cases, the user device 106 may be configured to acquire data from the wearable device 104 in response to connecting with the wearable device 104 (e.g., upon forming a wireless connection).

At 315, the user device 106 may perform activity segment identification on the data acquired from the wearable device 104. For example, the user device 106 may identify an activity segment during which the user is engaged in a physical activity. In some cases, the activity segment may be associated with activity segment data including at least the physiological data collected during the activity segment. The user device 106 may identify the activity segment based on acquired motion data, temperature data, or both. In such cases, the system 200 may use motion data and temperature data to identify activity segments. In some examples, the system 200 may identify the activity segment based on the motion data during the activity segment being greater than or equal to a motion threshold and based on a temperature drop during the activity segment being greater than or equal to a threshold temperature drop.

An activity segment may refer to a period of time during which a user is performing a physical activity. Activities may include any physical activity, such as exercises, sports, recreational activities, and physical work. Example activities may include, but are not limited to: "walking," "running," "cycling," "strength training," "high intensity interval training," "elliptical," "hiking," "swimming," "tennis," "rowing," "dance," "cross country skiing," "downhill skiing," "snowboarding," "golf," "hockey," "badminton," "horseback riding," "soccer," "yardwork," "stair stepper," "basketball," "squash," "house work," "volleyball," "surfing sports," and "skating sports."

Another example activity may include "other activity," which may act as a catchall for activities that are not defined. In some implementations, the activities may be grouped and/or categorized. In some cases, different groups/categories may be further defined by sub-groups/sub-categories. For example, a category/sub-category may include the category "winter sports" and the sub-category "skiing." In such cases, activities in the winter sports and skiing category/sub-category may include downhill skiing and cross country skiing. The user device 106 may also identify other user states in the acquired data which may include inactive states (e.g., resting, sitting, laying, etc.) and sleeping.

At 320, the user device 106 may send one or more activity segments to the server 110. For example, the user device 106 may send a current activity segment as the activity is occurring. The user device 106 may also send one or more past activity segments that may have already occurred and been completed. In some cases, each activity segment may be associated with an activity segment ID and/or time stamp data.

At 325, the server 110 may receive data from the user device 106 and perform activity classification operations based on the received data. The server 110 may receive any of the data described herein. For example, the server 110 may receive activity segment data for one or more activity segments. The server 110 may perform activity classification operations on each of the activity segments. In other words, the server 110 may be configured to determine one or more classified activity types for each respective activity segment.

In such cases, the system 200 may generate activity classification data associated with the activity segment based on the activity segment data. The activity classification data may include a plurality of classified activity types and corresponding confidence values. The confidence values may indicate a confidence level associated with the corresponding classified activity type. For example, upon receiving activity segment data for an identified activity segment at 320, the system 200 may generate activity classification data for the activity segment based on the activity segment data. In this regard, the system 200 may determine one or more classified activity types for the activity segment (e.g., identify the activity segment as a "running activity segment," a "swimming activity segment," or some other activity segment), and corresponding confidence values for each respective classified activity type.

In some examples, the system 200 may generate the activity classification data based on the motion data and the temperature data. For example, the system 200 may identify one or more motion features based on the motion data and identify one or more temperature features based on the temperature data. In such cases, generating the activity classification data may be based on the one or more motion features, the one or more temperature features, or both. In other words, the system 200 may be configured to differentiate between different classified activity types based on generated motion features, temperature features, or both. The one or more motion features may include an amount of motion during the activity segment. The one or more temperature features may include a temperature change during the activity segment, a rate of temperature change during the activity segment, or any combination thereof. Moreover, the system 200 may be configured to differentiate between different classified activity types based on other physiological parameters, including heart rate data, HRV data, respiratory rate data, blood oxygen saturation data, and the like.

In some implementations, the server 110 may receive historical activity data for the user. For example, the system 200 may identify historical activity segment data for the user. The historical activity segment data may include one or more historical activity segments for the user (e.g., previous time intervals in which the user was engaged in physical activities). In such cases, generating the activity classification data may be based on the historical activity segment data. For example, the system 200 may leverage historical activity data for the user to determine the activity types and/or confidence levels. For instance, if a user frequently goes on runs during the week, it may be more likely the current activity segment is also a "running activity segment." In some cases, the confidence values associated with the plurality of classified activity types may be based on the historical activity segment data. In other words, historical activity segment data may be used to "weight" or otherwise influence/adjust confidence values for classified activity types corresponding to subsequent activity segments.

In some aspects, the system 200 may be configured to perform activity classification (e.g., generate activity classification data) using a classifier or other machine learning model (e.g., machine learning classifier, random forest classifier, neural network, etc.). For example, the server 110 may be configured to input received activity segment data into a classifier or machine learning model, where the classifier/machine learning model is configured to generate the activity classification data (e.g., classified activity types, confidence values) based on the activity segment data. In some aspects, historical activity segment data may be used to train the classifier to improve activity classification techniques described herein. Moreover, in some aspects, user inputs received from a user (e.g., confirmation/rejection of classified activity types, modifications to activity segment data and/or activity classification data) may be used to further train the classifier to become more reliable and accurate with generating activity classification data.

At 330, the server 110 may send activity classification data to the user device 106. The activity classification data may include a plurality of classified activity types and associated confidence values.

At 335, the user device 106 (e.g., wearable application 250) may generate an activity GUI based on the received activity classification data. For example, the system 200 may cause a GUI of a user device 106 to display the activity segment data and at least one classified activity type of the plurality of classified activity types. The user device 106 may generate the activity GUI based on the confidence values associated with one or more of the activities. Example factors for generating the activity GUI may include, but are not limited to, the classified activity type associated with the highest confidence value, the highest confidence value relative to a threshold confidence value, the classified activity types associated with the highest two or more confidence values, the highest two or more confidence values relative to threshold values, and/or any classified activity types associated with confidence values that are greater than a minimum threshold confidence value.

The user device 106 (e.g., the wearable application 250) may modify the activity GUI based on the received confidence values. In some implementations, the user device 106 may modify the information/data displayed to the user in the activity GUI. For example, the user device 106 may modify the text (e.g., message to the user), graphical elements (e.g., images), and/or arrangement of the text/graphics included in the activity GUI. In some cases, the user device 106 may add or remove text/images.

For example, the system 200 may receive, via the user device 106 and in response to displaying the at least one classified activity type, one or more modifications for the activity segment. In other words, a user may be able to modify activity classification data displayed via the GUI 275. In such cases, causing the GUI to display the activity segment data may be based on receiving the one or more modifications. In some cases, the user may modify the activity segment (e.g., modify type of activity, time of activity, intensity of activity, and the like). In such cases, the one or more modifications may include an indication of an additional classified activity type associated with the activity segment.

For example, the user device 106 may modify activity GUI elements provided to the user. The user device 106 may modify the activity GUI interface elements, such as user input GUI elements (e.g., lists, menus, drop-down menus, buttons, etc.). The user device 106 may add or remove activity GUI elements. The different activity GUIs associated with different confidence value scenarios may be referred to as different modes or states. For example, the user device 106 may render an activity GUI in a first mode (or state) in response to determining a first confidence value for the activity GUI where a confidence value for an activity is very high. The user device 106 may render an activity GUI in a second mode (or state) in response to determining a second confidence value for the activity where there are multiple moderate confidence values for different activities.

At 340, the activity GUI may receive user input such as a confirmation that the activity is correct and/or a direct selection of the activity from a menu. For example, the system 200 may receiving, via the user device 106 and in response to displaying the at least one classified activity type, a confirmation of the activity segment. In such cases, causing the GUI to display the activity segment data may be based on receiving the confirmation. For example, the user may confirm the identified activity segment and verify "Yes, I completed the workout." In some cases, the confirmation may include a confirmation of the at least one classified activity type (e.g., "Yes, the workout was a running workout." In such cases, causing the GUI to display the activity segment data may be based on receiving the confirmation of the at least one classified activity type.

As noted previously herein, modifications to activity classification data and/or confirmations/denials of classified activity types may be used to further train classifiers and other models which are used to generate activity classification data based on received activity segment data.

The user-selected confirmation and/or classification may be stored in the user's historical activity history. Activity classification and activity GUI rendering described in the process flow 300 may be performed by a variety of computing devices described herein. In some implementations, the activity classification and/or activity GUI rendering may be performed in real time as data is acquired by a computing device (e.g., a ring). In other implementations, the activity classification and/or activity GUI rendering may be performed at other times, such as predetermined times and/or in response to user actions (e.g., opening the ring application).

In some aspects, each respective classified activity type may be associated with different parameters or characteristics, such as calorie consumptions, relative intensities, distances, paces, elevation gains, and the like. As such, in some aspects, the system 200 may adjust scores (e.g., Activity Scores, Readiness Scores) for the user based on determined classified activity types for each respective activity segment. For example, if a user changes a classified activity type for an activity segment from "hiking" to "elliptical," the system 200 may adjust/modify characteristics and parameters for the user, such as the user's daily Activity Score, calorie consumption, and the like.

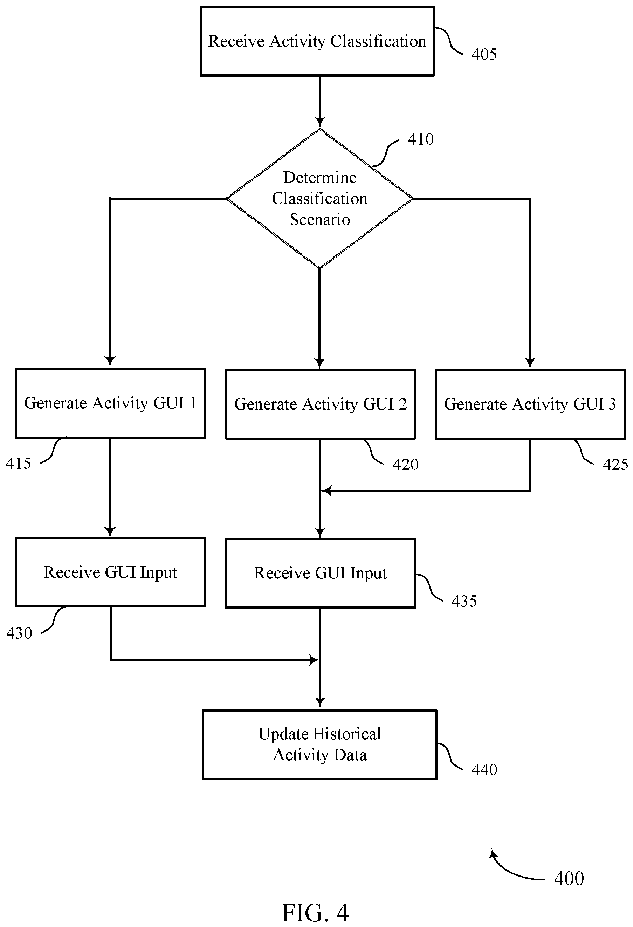

**FIG. 4** illustrates an example of a process flow 400 that supports activity classification and display in accordance with aspects of the present disclosure. The process flow 400 may be implemented by the system 200 including at least a server 110, a user device 106, a wearable device 104, or some combination of components from these devices. Alternative examples of the following may be implemented, where some steps are performed in a different order than described or not performed at all. In some cases, steps may include additional features not mentioned below, or further steps may be added. Process flow 400 may describe generation of activity GUIs based on received activity classification data.

At 405, the user device 106 may receive activity classification data from the server 110. At 410, the user device 106 may determine which activity GUI to generate based on the confidence values associated with the activities. In some implementations, the user device 106 may determine which activity GUI to generate based on receiving the activity classification.

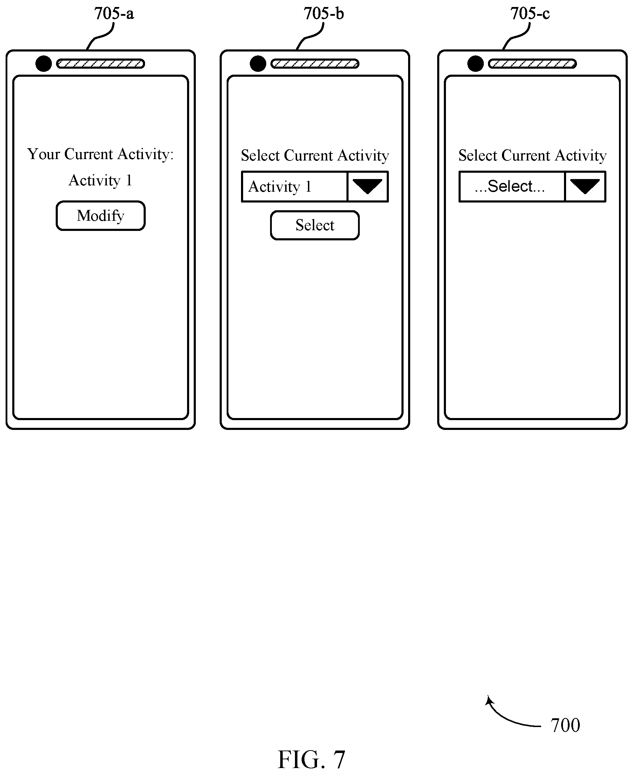

At 415, the user device 106 may generate a first activity GUI. For example, if the confidence values include a single high confidence value for a single activity, the user device 106 may render the first activity GUI, which may be further illustrated and described with reference to application page 705-a in FIG. 7. In some implementations, the first activity GUI may be generated based on determining which activity GUI to generate. Moreover, in some cases, the user device 106 may determine which activity GUI to generate based on the received activity classification data including the classified activity types and corresponding confidence values, as will be discussed in further detail herein.

At 430, the user device 106 may receive user input (e.g., using a modify button of the user device 106) to correct/modify the classified activity. In some implementations, the user device 106 may receive user input based on generating the first activity GUI.

At 420, the user device 106 may generate a second activity GUI. For example, if a single high confidence value may not be included in the activity classification data, the user device 106 may render the second activity GUI. In such cases, the user device 106 may generate the second activity GUI if the confidence values include moderate confidence values associated with multiple activities (e.g., 20-50% confidence values). The second activity GUI may be further illustrated and described with reference to application page 705-b in FIG. 7. In some implementations, the second activity GUI may be generated based on determining which activity GUI to generate.

At 435, the user device 106 may receive user input. In some implementations, the user device 106 may receive user input based on generating the second activity GUI. The user input may be an example of a user selection of the activity. For example, the user device 106 may receive a user selection of the activity.

At 425, the user device 106 may generate a third activity GUI. For example, if a single high confidence value may not be included in the activity classification data, the user device 106 may render the third activity GUI. In such cases, the user device 106 may generate the third activity GUI if the confidence values include low confidence values associated with multiple activities (e.g., less than a threshold confidence value). For example, each of the activities may be associated with a low confidence value. The third activity GUI may be further illustrated and described with reference to application page 705-c in FIG. 7. In some implementations, the third activity GUI may be generated based on determining which activity GUI to generate.

At 435, the user device 106 may receive user input. In some implementations, the user device 106 may receive user input based on generating the third activity GUI. The user input may be an example of a user selection of the activity. For example, the user device 106 may receive a user selection of the activity.

At 440, the user device 106 (e.g., the wearable application 250) may update historical activity data according to autoclassification and/or user classification of activities. In some implementations, the user device 106 may update the historical activity data based on receiving GUI input. In some examples, the user device 106 may update the historical activity data in response to receiving a user selection of the activity. In some aspects, updated historical activity data may be used to improve activity classification data for future activity segments (e.g., used to train the classifier used to generate activity classification data). Although the process flow may be described in the content of three GUIs, more or less than three GUIs for rendering the activity GUI and selecting activities may be used.

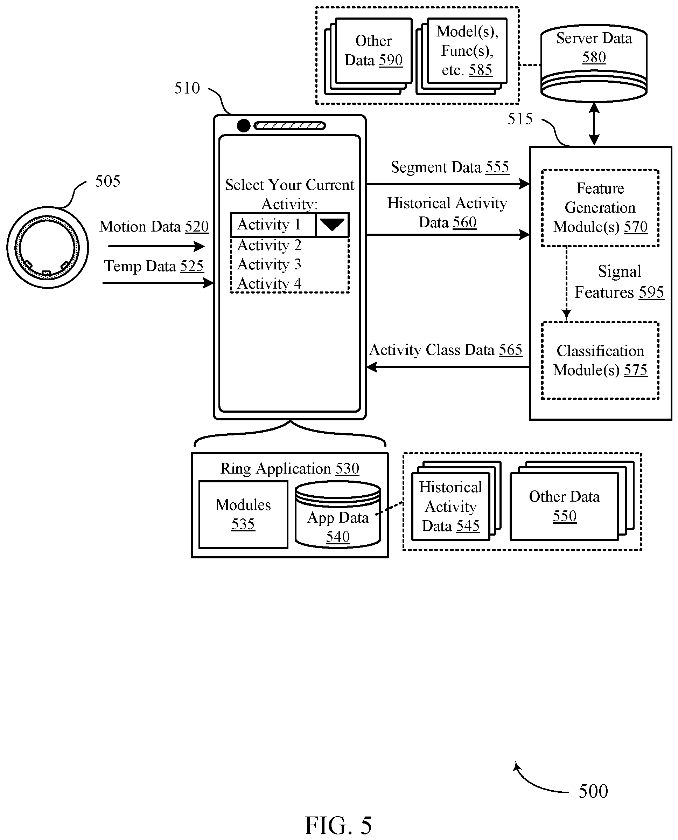

**FIG. 5** illustrates an example of a system 500 that supports activity classification and display in accordance with aspects of the present disclosure. The system 500 may implement, or be implemented by, system 100, system 200, or both. In particular, system 500 illustrates an example of a ring 505 (e.g., wearable device 104), a user device 510 (e.g., user device 106), and a server 515 (e.g., server 110), as described with reference to FIGs. 1 through 4.

The ring 505 may acquire motion data 520 and temperature data 525. In such cases, the ring 505 may transmit motion data 520 and temperature data 525 to the user device 510. The motion data 520 may include accelerometer data, gyro data, derived values of the accelerometer data and/or gyro data, or a combination thereof. The user device 106 may classify activities and generate activity GUIs based on the acquired data. In some cases, multiple devices may acquire physiological data. For example, a first computing device (e.g., user device 106) and a second computing device (e.g., the ring 505) may acquire motion data 520 and temperature data 525, respectively.

The user device 106 may include a ring application 530. The ring application 530 may include at least modules 535 and application data 540. In some cases, the application data 540 may include historical activity data 545 and other data 550. The other data 550 may include temperature data 525, motion data 520, or both.

The ring application 530 may present one or more classified activity types to the user for selection. The ring application 530 may modify which classified activity types are presented to the user for selection by the user (e.g., in a menu format). In some implementations, the ring application 530 may present a single classified activity type to the user if the activity has greater than a high threshold confidence value (e.g., greater than 90%). In some implementations, the ring application 530 may present multiple classified activity types to a user if the classified activity types each have greater than a threshold confidence value. In some implementations, the ring application 530 may remove classified activity types from selection that are associated with confidence values that are less than a threshold confidence value. For example, the ring application 530 may remove classified activity types from selection if the confidence values are near or equal to zero which may be the case for a majority of classified activity types when the selectable number of classified activity types is large. In some implementations, the activity GUI may rank the selectable classified activity types by associated confidence score (e.g., rank classified activity types from highest to lowest confidence value).

In some implementations, the ring application 530 may determine whether and/or when to render (e.g., display) the activity GUI elements described herein based on the activity classification data. For example, the ring application 530 may determine whether to show the activity GUI elements in an existing area of the ring application 530 based on the confidence values. In some examples, the ring application 530 may present the activity GUI elements to the user when the ring application 530 would benefit from user input that clarifies the current activity segment. For example, the ring application 530 may present the activity GUI elements when one or more of the confidence values are less than a threshold level of confidence and/or multiple classified activity types are associated with similar confidence values. In some examples, the ring application 530 may refrain from displaying the activity GUI when there is a high confidence level associated with the activity classification (e.g., greater than 90%).

In some implementations, the ring application 530 may notify the user of activity classifications and/or prompt the user to perform a variety of tasks in the activity GUI. For example, notifications may notify the user of a recently classified activity segment. In some examples, a prompt may request classification and/or confirmation by the user. The notifications and prompts may include text, graphics, and/or other user interface elements. The notifications and prompts may be included in the ring application 530 such as when there is an activity segment that has just been classified (e.g., a detected workout/activity segment which has been classified into one or more classified activity types), the ring application 530 may display notifications and prompts. The user device 510 may display notifications and prompts in a separate window on the home screen and/or overlaid onto other screens (e.g., at the very top of the home screen). In some cases, the user device 510 may display the notifications and prompts on a mobile device, a user's watch device, or both.

In some implementations, the ring application 530 may automatically classify an activity segment. For example, the ring application 530 may automatically classify an activity segment if the confidence value associated with a classified activity type is greater than a high threshold value. In such cases, the ring application 530 may provide an activity GUI element for the user to change the automatically classified activity if the automatically classification is incorrect.

In some implementations, the user device 510 may store historical user data. In some cases, the historical user data may include historical activity data 545. The historical activity data 545 may include a list of activities performed by the user, data indicating when the activities were performed, or both. In some examples, historical activity data 545 may include activity and timestamp pairs for a period of time (e.g., a past number of months). The historical activity data 545 may be used (e.g., by the user device 510 or server 515) to determine a number of times a user performed an activity, a frequency of specific activities, the common times of day a user performs specific activities, or a combination thereof. For example, if a user has walked 10 times and ran 5 times, the frequency of walking is 0.66 (e.g., ten times walking divided by fifteen total activities), and the frequency of running is 0.33 (e.g., five times running divided by fifteen total activities). The user device 106 and/or server 515 may calculate data for each classified activity type (e.g., frequencies for each classified activity type). The historical activity data 545 and other data 550 (e.g., frequency data) associated with the historical activity data 545 may be used by the server 515 (e.g., classification module 575) to classify activities for the user. Using the historical activity data 545 may allow the user device 106 and/or server 515 to personalize the activity classification and activity GUI by taking into consideration the preferred user activities.

The user device 510 may transmit segment data 555 and historical activity data 560 to the server 515. In some cases, the transmitted historical activity data 560 may be the same historical activity data 545 stored in the ring application 530. In other examples, the historical activity data 560 may be different than the historical activity data 545 stored in the ring application 530. The server 515 may receive the segment data 555 and the historical activity data 560. The segment data 555 may include segment motion data, segment temperature data, or both.

In some cases, the server 515 may generate a plurality of historical activity features (e.g., signal features 595) for the user via the feature generation module 570. The historical activity features may include numbers that indicate how many times each classified activity type was performed and/or a frequency associated with the classified activity types. For example, the feature generation module 570 may generated a signal feature 595 for each classified activity type that indicates how many times the classified activity type was performed and/or a frequency at which the classified activity type was performed. In some cases, the historical activity features may include the durations for which the classified activity type were performed. In some examples, the historical activity features may include when the classified activity type were performed (e.g., a most common time period), such as a time of day, part of the day (e.g., morning, afternoon, night), day of the week, and/or a time of the year. The historical activity features may help the classification module 575 (e.g., a machine learned model) determine which activities (e.g., classified activity types) the user avoids, performs, and prefers.

As described herein, the user-specified classifications and/or automatic classifications presented in an activity GUI may be included in the historical activity data to be used in future classifications (e.g., as scoring features). Although a general classification model may be used across a plurality of users, the historical activity features may help personalize the output of the classification model according to the user's specific activities.

The server 515 (e.g., one or more feature generation modules 570) may generate a signal feature 595 for each activity segment (e.g., segment data 555). The signal features 595 may include physiological data features, historical activity features, or both. The physiological data features may include motion features, temperature features, or other physiological data features determined from other physiological data, such as heart rate features, HRV features, and respiratory rate features. In some implementations, the server 515 may determine a plurality of statistical features for each set of data received from the user device 510.

In some cases, the feature generation module 570 of the server 515 may generate one or more motion features (e.g., signal features 595) for segment data 555. The motion features may include accelerometer (acceleration) features, gyro features, and derived value features for one or more axes. The accelerometer features may include statistical features for one or more axes, such as minimum values, maximum values, average values, delta values, median values, variance values, sums, deviations (e.g., mean absolute deviations), standard error of the mean, skew, absolute energy, and other statistical values. In some cases, the server 515 may determine one or more gyro features for one or more axes. The server 515 may determine one or more derived value features based on any of the derived values, such as motion count values, regularity values, intensity values, METs, and orientation values.

The feature generation modules 570 of the server 515 may generate one or more temperature features for segment data 555. Using the temperature features may improve the accuracy of the activity classification. The temperature features may include statistical features, such as minimum values, maximum values, average values, delta values, median values, variance values, sums, deviations (e.g., mean absolute deviations), standard error of the mean, skew, absolute energy, and other statistical values. In some implementations, temperature features may include one or more temperature drop features, such as features that may be based on a drop in temperature between two points in time. The temperature drop features may be in absolute units (e.g., degrees Celsius) or relative units (e.g., drop relative to a max value). One or more temperature drop features may be calculated between any two points in the segment data 555, such as between the start-end points, max-min points, start-min points, or other points.

The classification modules 575 of the server 515 may classify the segment data 555 as being associated with one or more activities (e.g., classified activity types) based on the received temperature features, motion features, and historical activity features (e.g., signal features 595). For example, the classification modules 575 may generate an output that includes a plurality of classified activity types, each of which may be associated with a confidence value that indicates a level of confidence associated with the respective classified activity type. In such cases, the server 515 may output the activity class data 565 to the user device 510. The classification modules 575 may generate a confidence value for each classified activity type. For example, for a single segment data 555, the classification modules 575 may be configured to output a confidence value for each of a plurality of classified activity types.

The confidence values for each classified activity type may be a number (e.g., a decimal number) from 0.00-1.00 that indicates a level of confidence that the segment data 555 is associated with the classified activity type. In some implementations, a confidence value closer to 0.00 may indicate a lower level of confidence in the classified activity type. In other examples, a confidence value closer to 1.00 may indicate a higher level of confidence in the classified activity type. In some implementations, the confidence value may be interpreted as a probability score that indicates a probability that the segment data 555 is associated with an activity. For example, a confidence score of 0.50 may indicate a 50% probability (e.g., confidence) that the determined classified activity type for the activity segment is accurate. In some implementations, the sum of the confidence values across all activity outputs may be equal to 1.00.

In some implementations, the user device 510 and/or server 515 may also store other data 590 which may be an example of user information. The user information may include, but is not limited to, user age, weight, height, and gender. In some implementations, the user information may be used as features for the classification modules 575. The server data 580 may include the other data 590 and the modules and functions 585.

The automatic activity classifications and/or user-specified activity classifications may be used by one or more computing devices in a variety of ways. In some implementations, the activity classifications may be stored as a user's historical activity data 545 which may then be used in further activity classifications (e.g., to personalize future classifications). The activity classifications may also be used to generate reports/metrics for activity and exercise tracking such as training logs and calorie counting. In some cases, the activity classifications may be used to generate reports/metrics associated with rest and recovery. In some implementations, the activity classifications may be used for personalized health guidance and recommendations.

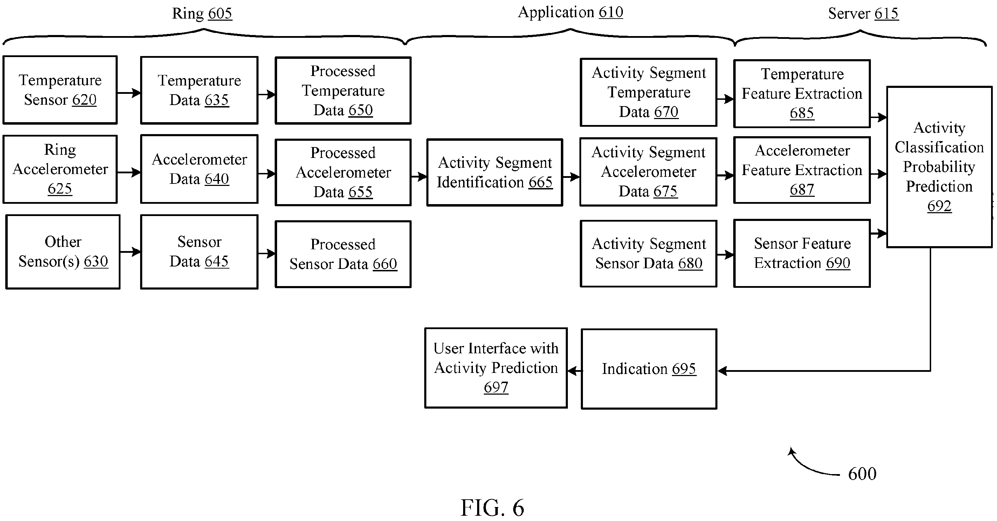

**FIG. 6** illustrates an example of a system 600 that supports activity classification and display in accordance with aspects of the present disclosure. The system 600 may implement, or be implemented by, system 100, system 200, system 500, or a combination thereof. In particular, system 600 illustrates an example of a ring 605 (e.g., wearable device 104), an application 610 (e.g., user device 106), and a server 615 (e.g., server 110), as described with reference to FIGs. 1 through 5.

The ring 605 may include at least a temperature sensor 620, a ring accelerometer 625, and other sensors 630. In some implementations, the ring 605 may acquire and process raw motion data (e.g., accelerometer data 640) and raw temperature data 635. The accelerometer data 640 and temperature data 635 may include sampled values. In some cases, Accelerometer data 640 may include motion data and gyro data. For example, accelerometer data 640 may include accelerometer values for multiple axes, such as an X, Y, and Z axis. Temperature data 635 may include temperature values sampled from one or more temperature sensors 620. Additionally, or alternatively, raw data (e.g., temperature data 635 and/or accelerometer data 640) may be acquired from other devices, such as a mobile device.

In such cases, the temperature sensor 620 may determine temperature data 635. The temperature data 635 may include minimum temperature values, maximum temperature values, average temperature values, delta temperature values, median temperature values, variance temperature values, temperature sums, temperature deviations (e.g., mean absolute deviations), standard error of the mean, skew, absolute energy, and other statistical temperature values. In some cases, the temperature data 635 may include temperature decrease/increase values from a baseline temperature. The baseline temperature may be an average temperature over a prior period of time (e.g., over one or more previous time windows) such as a period of time on the order of minutes to hours. The temperature data 635 may include temperature changes (e.g., temperature drops or increases) and temperature drop speed (e.g., rate). For example, a temperature relative drop may be calculated as (temp_max - temp_min)/temp_max. In other examples, temperature drop speed may be calculated as (temp_max - temp_min)/activity_duration. The temperature values may be calculated in degrees Celsius or as relative values.

The ring accelerometer 625 may determine other motion values over time, such as minimum motion values, maximum motion values, other average values, delta values, median values, variance values, sums, deviations (e.g., mean absolute deviations), standard error of the mean, skew, absolute energy, and other statistical values. In some cases, the ring accelerometer 625 may determine acceleration values and gyro values based on multiple axis of motion, such as acceleration values over time based on X, Y, and Z axis.

In some cases, the ring 605 may determine one or more derived values from the raw motion data (e.g., accelerometer data 640) and/or the raw temperature data 635. The derived values for accelerometer data 640 may include, but are not limited to, motion count values, regularity values, intensity values, METs, and orientation values. The ring 605 may calculate each of the derived values over set periods of time, such as each 30 second interval, 1 minute interval, or other intervals.

In some implementations, the ring 605 (e.g., other sensors 630) may acquire other raw physiological data in addition to accelerometer data 640 and temperature data 635. The other sensors 630 may determine additional values based on the additional physiological data. For example, the other sensors 630 may determine heart rate data, HRV data, respiratory rate data, blood oxygen saturation data, and other physiological parameters based on the additional physiological data. The other sensors 630 may process the additional physiological data (e.g., sensor data 645) and generate values (e.g., average values, max/min values, etc.) for the additional data over set periods of time, such as every 30 second interval or 1 minute interval.

The ring 605 may include processed temperature data 650, processed accelerometer data 655, and processed sensor data 660. Any of the processed temperature data 650, processed accelerometer data 655, or other processed sensor data 660 described herein may be calculated by computing devices other than the ring 605. For example, the processed temperature data 650, processed accelerometer data 655, and processed sensor data 660 may be determined by the user device (e.g., application 610), server 615, or other computing device (e.g., a watch or personal computing device). In some cases, any of the processed temperature data 650, processed accelerometer data 655, and processed sensor data 660 may be used as input (e.g., features) for classifying a user's current or prior activities. The time periods over which the processed temperature data 650, processed accelerometer data 655, and processed sensor data 660 are determined may be similar to one another, or may vary, depending on the type of calculation and data used for the calculation. Accordingly, the processed temperature data 650, processed accelerometer data 655, and processed sensor data 660 may be calculated over time period of seconds, minutes, hours, or longer, depending on the calculation.

The application 610 (e.g., wearable application 250 implemented by the user device 106 in FIG. 2) may perform activity segment identification 665 based on sampling the processed accelerometer data 655. The application 610 may generate activity segment temperature data 670, activity segment accelerometer data 675, and activity segment sensor data 680.

The server 615 may classify each of the identified activity segments (e.g., activity segment temperature data 670, activity segment accelerometer data 675, and activity segment sensor data 680). For example, the server 615 may generate segment features for each segment. In such cases, the server 615 may generate temperature feature extraction 685, accelerometer feature extraction 687, and sensor feature extraction 690. The server 615 may classify the segments based on the generated segment features. For example, the server 615 may input the temperature feature extraction 685, accelerometer feature extraction 687, and sensor feature extraction 690 into the activity classification probability prediction 692. In some implementations, the server 615 may use one or more machine learned models that were trained using features described herein for a plurality of users over time (e.g., motion features, temperature features, and historical activity features). Although the classification operations described herein may use one or more machine learned models, the server 615 may classify a segment using a variety of other techniques, such as rule based algorithms, functions (e.g., weighted functions), and/or other models.

In some implementations, temperature features (e.g., temperature feature extraction 685) may include one or more temperature rate features (e.g., temperature drop rate features). The temperature rate features may indicate a change in temperature between any two points in time during the segment. In some implementations, temperature rate features may include temperature drop rate features that indicate the amount the user's temperature decreases over a period of time. One or more temperature drop rate features may be calculated between any two points in the segment, such as between adjacent points, the start-end points, max-min points, start-min points, or other points. The temperature rate features may also include temperature rise rate features (e.g., increase rate features) that indicate the amount the user's temperature has increased over a period of time. One or more temperature rise rate features may be calculated between any two points in the segment, such as between adjacent points, the start-end points, max-min points, start-min points, min-end points, or other points. The temperature rate features may be in absolute units (e.g., degrees Celsius) or in relative units (e.g., temperature drop/rise relative to a baseline). In some examples, temperature feature extraction 685 may include a temperature drop/rise during a period of time (e.g., during 1 minute). In some cases, temperature relative drop may equal (temp_max - temp_min)/temp_max. The temperature drop rate (e.g., speed) may be equal to (temp_max - temp_min)/activity_duration. In other examples, a temperature feature extraction 685 may include a maximum drop value and/or average drop value during the entire activity or during a period of time.

In some implementations, the temperature feature extraction 685, and accelerometer feature extraction 687 may be binary features (e.g., 0/1) that indicate whether temperature conditions and/or motion conditions have been satisfied. For example, a temperature feature extraction 685 may indicate whether (e.g., via 0 or 1) temperature has dropped greater than a threshold amount (e.g., within a period of time). In some cases, a temperature feature extraction 685 may include a drop rate of X degrees per minute for a time window (e.g., a time window of 1-10 minutes in duration). In some implementations, accelerometer feature extraction 687 may indicate whether (e.g., 0/1) a threshold amount of motion (e.g., acceleration) has been detected within the segment.

Although temperature data 635, accelerometer data 640, and other sensor data 645 may each be used alone to generate respective features, in some implementations, features may be generated based on multiple data types. For example, a motion-temperature feature may be generated based on temperature data 635 and accelerometer data 640. In some cases, a feature may indicate whether (e.g., 0/1) temperature has decreased while motion has increased (e.g., for a period of time).

The application 610 may generate an indication 695 based on the activity classification probability prediction 692. In some cases, the application 610 may display the indication 695 via the user interface with activity prediction 697.

**FIG. 7** illustrates an example of a GUI 700 that supports activity classification and display in accordance with aspects of the present disclosure. The GUI 700 may implement, or be implemented by, aspects of the system 100, system 200, process flow 300, process flow 400, system 500, system 600, or any combination thereof. For example, the GUI 700 may be an example of a GUI 275 of a user device 106 (e.g., user device 106-a, 106-b, 106-c) corresponding to a user 102.

In some examples, the GUI 700 illustrates a series of application pages 705 which may be displayed to a user 102 via the GUI 700 (e.g., GUI 275 illustrated in FIG. 2). The GUI 700 may be an example of an activity GUI. The GUI 700 may be generated on the user device 106. In some implementations, the GUI 700 may be generated by the wearable application 250. In other implementations, the GUI 700 may be a web-based activity GUI (e.g., provided by the server 110). Although the GUI 700 is illustrated on a mobile user device 106, the GUI 700 may be generated on other computing devices using other applications and/or web-based interfaces. The GUI 700 may be an example GUI that includes example text, images, and activity GUI elements that may be included in the GUI 700. As such, other activity GUIs that are not explicitly illustrated herein may be generated according to the present disclosure.

In some implementations, the wearable application 250 may generate the GUI 700 based on the received activity classification data. For example, the wearable application 250 may generate the GUI 700 based on the confidence values associated with the classified activity types. In such cases, application pages 705 may be rendered by the ring application based on different confidence values.

The application page 705-a may be an example of a GUI 700 that specifies a user's current classified activity type (e.g., "Activity 1"). The application page 705-a may include a modify GUI element (e.g., a modify button) that the user may select (e.g., touch/click) in order to modify the classified activity type. For example, selecting the modify button may cause the ring application to present a list of possible classified activity types to the user for selection. The list of possible classified activity types may be ranked by corresponding confidence values. The application page 705-a may be rendered if a classified activity type is associated with a high confidence value (e.g., greater than 90%) as the classified activity type may be automatically chosen for the user.

The application page 705-b may be an example of a GUI 700 instructs the user to select their current activity (e.g., current activity type). An activity type may be provided to the user in a menu GUI element (e.g., a drop down menu). The user may select the "Select" activity GUI element to select the provided activity type. In some cases, the user may select (e.g., touch/click) the menu GUI element to view one or more additional possible activities. The application page 705-b may be rendered if moderate confidence values may be associated with multiple activities. In such cases, a highest ranking activity (e.g., a highest confidence value) may be placed at the top of the menu. In some cases, including the "Select" button may prompt the user to verify which of the classified activity types they are performing as the confidence values may not allow for reliable automatic classification.

Application page 705-c may be an example of a GUI 700 that instructs the user to select their current activity. A menu GUI element may be rendered, but an activity may not be automatically rendered for selection. Instead, the user may be prompted by the menu GUI element to interact with the menu GUI element in order to select a current activity type. The application page 705-c may be rendered if the classification data does not include a classified activity type associated with a high level of confidence. For example, each of the classified activity types may have less than a threshold level of confidence. In such cases, user selection of the classified activity type may be preferred for an accurate activity classification.

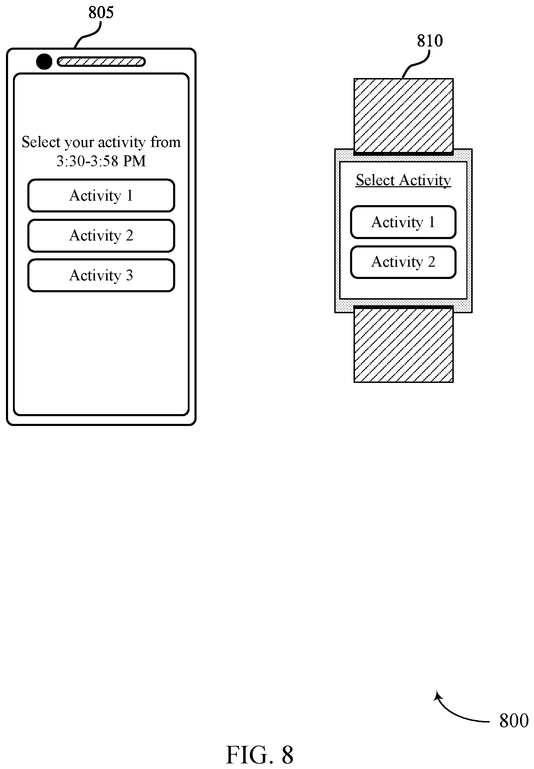

**FIG. 8** illustrates an example of a GUI 800 that supports activity classification and display in accordance with aspects of the present disclosure. The GUI 800 may implement, or be implemented by, aspects of the system 100, system 200, process flow 300, process flow 400, system 500, system 600, or any combination thereof. For example, the GUI 800 may be an example of a GUI 275 of a user device 106 (e.g., user device 106-a, 106-b, 106-c) corresponding to a user 102, a GUI 700, or both.

In some examples, the GUI 800 illustrates a series of application pages 805 which may be displayed to a user 102 via the GUI 800 (e.g., GUI 275 illustrated in FIG. 2). The GUI 800 may be an example of an activity GUI. For example, the application page 805-a may be generated on the user device 106. In some implementations, the GUI 800 may be generated by the wearable application 250. In other implementations, the GUI 800 may be a web-based activity GUI (e.g., provided by the server 110). Although the GUI 800 is illustrated on a mobile user device, the GUI 800 may be generated on other computing devices using other applications and/or web-based interfaces. The GUI 800 may be an example GUI that includes example text, images, and activity GUI elements that may be included in the GUI 800. As such, other activity GUIs that are not explicitly illustrated herein may be generated according to the present disclosure.

In some implementations, the wearable application 250 may generate the GUI 800 based on the received activity classification data. For example, the wearable application 250 may generate the GUI 800 based on the confidence values associated with the classified activity types. In such cases, application pages 805 may be rendered by the wearable application 250 based on different confidence values.

The application page 805-a may be an example of a GUI 800 that instructs the user to select an activity (e.g., classified activity type) they performed at an earlier time (e.g., 3:30-3:58 PM of the same calendar day). In some cases, application page 805-a may provide a user with the ability to classify prior activity segments (e.g., prior to a current time). In some cases, the application page 805-a may include multiple selection GUI elements for selecting a specific prior activity segment. The user may select (e.g., touch/click) the activity button corresponding to the prior activity segment. The application page 805-a may be rendered such that there may be multiple classified activity types (e.g., three activities) associated with confidence levels that may not provide a great level of certainty (e.g., 20-30%) as to the exact activity that was performed. In application page 805-a, the activity buttons may be ranked by confidence value associated with the classified activity type. For example, the application page 805-a may display "Activity 1" at the top of the list as the activity type may be the highest ranking activity (e.g., the classified activity type with the highest confidence value).

The application page 805-b may be displayed on a watch computing device. The application page 805-b may operate in a similar manner as the application page 805-a. In some implementations, the watch computing device may be used instead of the user device 106 (e.g., a mobile device). In such cases, the watch computing device may acquire data from the ring, send segment data to the server 110, receive activity classification data from the server 110, and execute an application that renders the application page 805-b.

Instead of replacing the user device 106 that displays application page 805-a, the watch computing device may be used as an additional computing device. For example, a user may be associated with a ring, a first user device (e.g., a mobile device), and a second user device (e.g. a watch computing device). The first user device may acquire data from the ring, send segment data to the server 110, and receive activity classification data from the server 110. The wearable application 250 may execute on the first user device (e.g., a mobile device) and the second user device (e.g., the watch computing device).

The first user device may send activity data (e.g., activities, confidence values, and/or activity GUI data) to the second computing device. The second computing device may notify the user via application page 805-b (e.g., by a vibration/sound), that the user should select a classified activity type. Selection of the classified activity type on the second user device may be communicated to the first user device for storage in the historical activity data. Although the first user device and second user device may be described as a mobile device and a watch computing device, any combination of computing devices may be used (e.g., a tablet, head-mounted device, laptop, etc.).

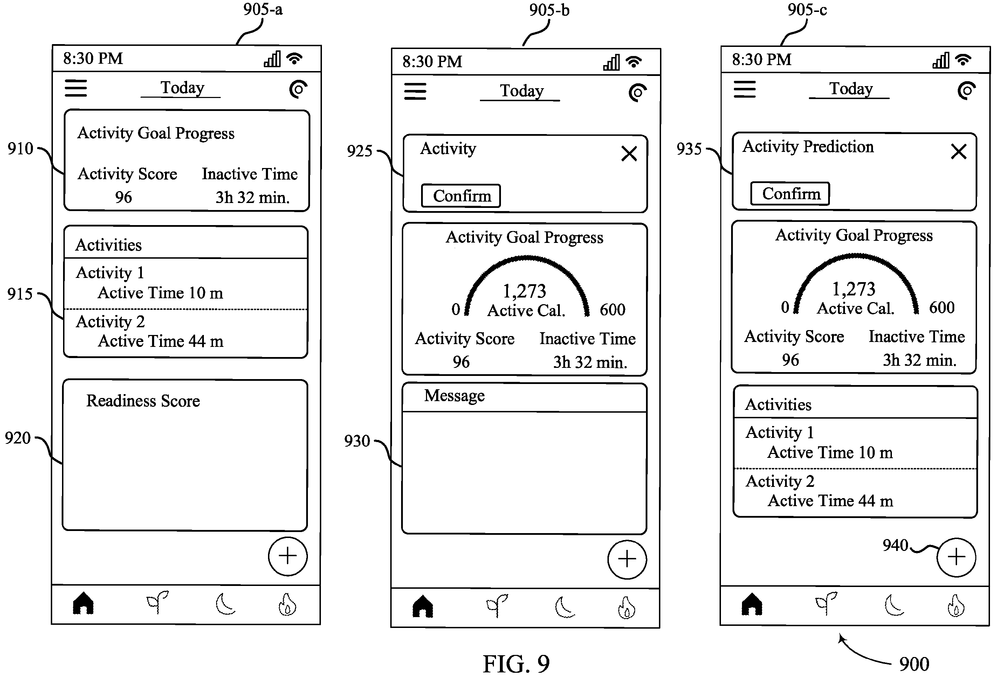

**FIG. 9** illustrates an example of a GUI 900 that supports activity classification and display in accordance with aspects of the present disclosure. The GUI 900 may implement, or be implemented by, aspects of the system 100, system 200, process flow 300, process flow 400, system 500, system 600, or any combination thereof. For example, the GUI 900 may be an example of a GUI 275 of a user device 106 (e.g., user device 106-a, 106-b, 106-c) corresponding to a user 102, a GUI 700, a GUI 800, or a combination thereof.

In some examples, the GUI 900 illustrates a series of application pages 905 which may be displayed to a user 102 via the GUI 800 (e.g., GUI 275 illustrated in FIG. 2). The GUI 900 may be an example of an activity GUI. For example, the application pages 905 may be generated on the user device 106. In some implementations, the GUI 900 may be generated by the wearable application 250. In other implementations, the GUI 900 may be a web-based activity GUI (e.g., provided by the server 110). Although the GUI 900 is illustrated on a mobile user device, the GUI 900 may be generated on other computing devices using other applications and/or web-based interfaces. The GUI 900 may be an example GUI that includes example text, images, and activity GUI elements that may be included in the GUI 900. As such, other activity GUIs that are not explicitly illustrated herein may be generated according to the present disclosure.

In some implementations, the wearable application 250 may generate the GUI 900 based on the received activity classification data. For example, the wearable application 250 may generate the GUI 900 based on the confidence values associated with the classified activity types. In such cases, application pages 905 may be rendered by the wearable application 250 based on different confidence value.

The application page 905-a may display an activity goal progress card 910, an activity list 915, and a Readiness Score 920. In such cases, the application page 905-a may display activity segment data and activity GUI elements. In some examples, the user may select an activity segment within the activity list 915. Each activity segment may be associated with a single activity card interface element. In some cases, each activity card may include an activity name, activity timestamp, activity duration, activity calorie burn, and confidence value. The user may scroll through a plurality of activity GUIs (e.g., activity cards such the activity goal progress card 910, the activity list 915, etc.) displayed via the application page 905-a. For example, the user may swipe up or down on the application page 905-a to scroll through historical activity cards.

The activity list 915 may include one or more activity cards corresponding to respective activity segments. The information included in the activity cards may be based on the confidence values associated with classified activity types for each respective activity segment. In some cases, the classified activity types included in the activity list 915 may be automatically classified and added the user's historical activity data. The user may select an activity segment from the activity list 915, and the system may generate an expanded view of the activity segment with additional information (e.g., physiological parameters associated with the activity segment). The user may also modify the classified activity type in the expanded view. In some cases, the activity cards of the activity list 915 may be generated if the confidence values associated with the classified activity type are high.

In some cases, an activity goal progress card 910 may be displayed to the user via the GUI 900 of the user device 106 that indicates the activity score and the inactive time. The activity goal progress card 910 may include an active calorie burn count, an active time, or both. The Readiness Score 920 may be updated based on identified activity segments and corresponding classified activity tyeps. Additionally, in some implementations, the application page 905-a may display one or more scores (e.g., Sleep Score, Readiness Score 920, Activity Score) for the user for the respective day.

The application page 905-b may display the message 930 and the activity confirmation card 925. For example, the system may generate the activity confirmation card 925. The activity confirmation card 925 may include a confirmation GUI element that the user may select in order to confirm the activity segment and/or classified activity type. In such cases, the application page 905-b may display the activity confirmation card 925 that indicates that the activity segment has been recorded. In some implementations, upon confirming the activity confirmation card 925 is valid, the activity segment may be recorded/logged in an activity log for the user 102 for the respective calendar day. Moreover, in some cases, the activity segment may be used to update (e.g., modify) one or more scores associated with the user 102 (e.g., Activity Score, Readiness Score 920). That is, data associated with the identified activity segment may be used to update the scores for the user 102 for the following calendar day after which the activity segment was confirmed. In some cases, the messages 930 displayed to the user via the GUI 900 of the user device 106 may indicate how the activity segment affected the overall scores (e.g., overall Activity Score, overall Readiness Score 920) and/or the individual contributing factors.

In some cases where the user 102 dismisses the prompt (e.g., activity confirmation card 925) on application page 905-b, the activity confirmation card 925 may disappear, and the user may input an activity segment via input 940 at a later time. The server of system 200 may receive user input, via input 940, information associated with the activity segment. In such cases where the user 102 dismisses the activity segment, the activity segment may be removed from the user's historical activity. In other examples, the user 102 may edit the activity confirmation card 925 to modify the activity segment by updating the activity name, classified activity type, activity timestamp, activity duration, intensity, or a combination thereof. In some cases, the user 102 may select a different activity segment.

The user 102 may receive activity confirmation card 925, which may prompt the user 102 to verify whether the activity segment has occurred or dismiss the activity confirmation card 925 if the activity segment has not occurred. In such cases, the application page 905-b may prompt the user to confirm or dismiss the activity segment (e.g., confirm/deny whether the system 200 correctly determined that the user 102 was engaged in physical activity during the identified activity segment). For example, the system 200 may receive, via the user device 106 and in response to predicting the activity segment, a confirmation of the activity segment.

In some cases, confirming and/or denying whether the system 200 correctly determined that the user 102 was engaged in physical activity during the activity segment may update the confidence value associated with the activity segment. In some cases, a classified activity type for an activity segment may be associated with the highest probability of the classified activity types, but not high enough for confident autoclassification.

The user 102 may receive activity prediction card 935, which may prompt the user 102 to verify whether the activity segment has occurred or dismiss the activity prediction card 935 if the activity segment has not occurred (e.g., confirm or deny whether the user was engaged in physical activity during the activity segment). In such cases, the application page 905-c may prompt the user to confirm or dismiss the predicted activity segment (e.g., confirm/deny whether the system 200 correctly determined that the user 102 experienced the activity segment). For example, the system 200 may receive, via the user device 106 and in response to identifying the activity segment, a confirmation of the activity segment. The activity prediction card 935 may indicate a level of uncertainty for the activity segment identification. For example, the activity prediction card 935 may display a "Maybe Activity" to indicate that the system 200 identified that the user may have been engaged in physical activity during the potential activity segment. In such cases, whether the user confirms or denies the activity segment may affect the confidence value. In some cases, the confidence value of a classified activity type displayed via activity prediction card 935 may be lower than the confidence value of the classified activity type displayed via activity confirmation card 925.

In some cases, the user 102 may log symptoms via input 940. For example, the system 200 may receive user input (e.g., tags) to log symptoms associated with the activity segment or the like (e.g., cramps, headaches, pain, windy, hot, etc.). The system 200 may recommend tags to the user 102 based on user history and the activity segment.

In some implementations, the system 200 may be configured to receive user inputs regarding detected/predicted activity segments in order to train classifiers (e.g., supervised learning for a machine learning classifier) and improve activity prediction techniques. For example, the user device 106 may display an identified activity segment. Subsequently, the user 102 may input one or more user inputs, such as a beginning time of the activity segment, a confirmation of the activity segment, a confirmation of the predicted classified activity type for the activity segment, and the like. These user inputs may then be input into the classifier to train the classifier. In other words, the user inputs may be used to validate, or confirm, predicted activity segments.

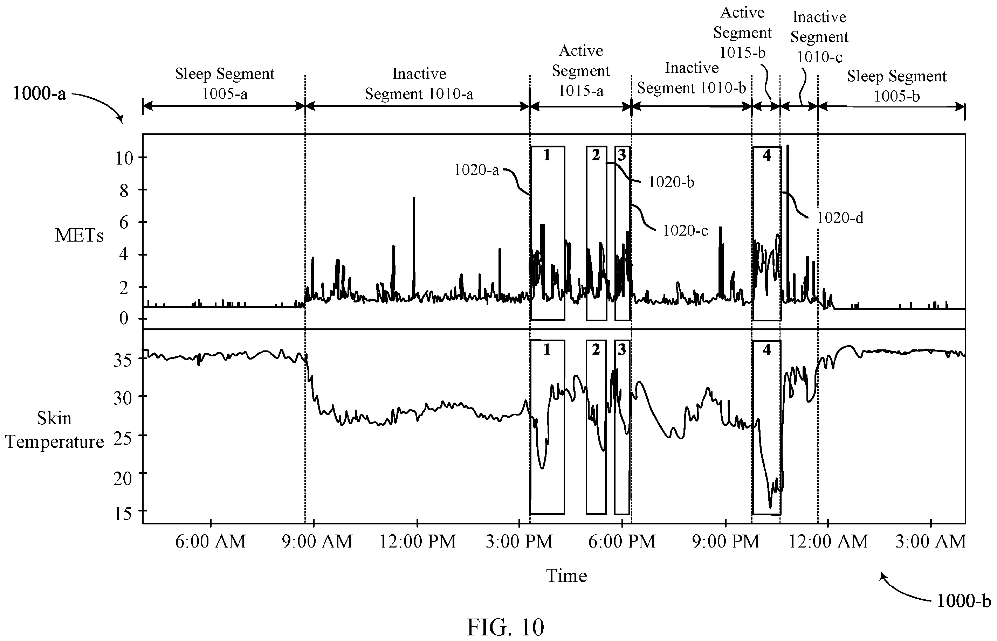

**FIG. 10** illustrates an example of a activity segment classification diagram 1000 that supports activity classification and display in accordance with aspects of the present disclosure. The activity segment classification diagram 1000 may implement, or be implemented by, aspects of the system 100, system 200, or both. For example, in some implementations, the activity segment classification diagram 1000 indicates a relative timing of sleep segments 1005, an inactive segments 1010, and active segments 1015. The activity segment classification diagram 1000 may be displayed to a user via the GUI 275 of the user device 106, GUI 900, or both as shown in FIGs. 2 and 9.

As will be described in further detail herein, the system 200 may be configured to detect a sleep segment 1005, an inactive segment 1010, and/or an active segment 1015 for a user 102. As such, the activity segment classification diagram 1000-a illustrates a relationship between a user's motion data and the sleep segments 1005, inactive segments 1010, and/or active segments 1015. As shown in activity segment classification diagram 1000-a, motion data may be represented as metabolic equivalents (METs). The activity segment classification diagram 1000-b illustrates a relationship between a user's temperature data and the sleep segments 1005, inactive segments 1010, and/or active segments 1015. In some cases, the system 200 may determine, or estimate, sleep segments 1005, inactive segments 1010, and/or active segments 1015for a user 102 based on motion data, temperature data, or both, for the user collected via the ring (e.g., wearable device 104).

In particular, as described herein, the system 200 may identify one or more activity segments 1020 during which the user is engaged in physical activity within the active segments 1015. In other words, the system 200 may generally identify or flag intervals of time in which the user exhibits heightened activity as "activity segments," and may identify sub-sets of active segments 1015 as "activity segments 1020" during which the user is engaged in physical activity (e.g., activity segments 1020 when the user is engaged in a workout or other exercise). In some aspects, activity segments 1020 may be identified based on motion data, temperature data, or both. Moreover, as described previously herein, additional or alternative physiological parameters may be used to identify activity segments in which the user may be engaged in physical activity.

The activity segment classification diagrams 1000 shown in FIG. 10 illustrates a relative timing of the sleep segments 1005, inactive segments 1010, and/or active segments 1015 relative to traditional calendar days. In particular, the activity segment classification diagrams 1000 illustrates the sleep segments 1005, inactive segments 1010, and/or active segments 1015 for a user for a single calendar day (e.g., from at least 6:00 AM to at least 3:00 AM).

Activity segment classification diagrams 1000 may include one or more sleep segments 1005, inactive segments 1010, and active segments 1015. For example, the activity segment classification diagrams 1000 may include sleep segments 1005-a and 100-b, inactive segments 1010-a, 1010-b, and 1010-c, and active segments 1015-a and 1015-b. The active segment 1015-a may include at least three identified activity segments 1020-a, 1020-b, and 1020-c, and the active segment 1015-b may include at least one identified activity segment 1020-d. The sleep segments 1005 may occur at both ends of the time series. In some case, between sleep segment 1005-a and active segment 1015-a, the system 200 may detect an inactive segment 1010-a. The sleep segments 1005, inactive segments 1010, and active segments 1015 may be determined based on MET values, temperature values, and/or other values (e.g., other motion values).

The data acquired by the user device 106 may include one or more active segments 1015. For example, if the user device 106 receives data from the ring (e.g., wearable device 104) as the data is generated (e.g., every 30 seconds to 1 minute), the user device 106 may receive data while the user is performing an activity. In such cases, the user device 106 may generate time series data for a current active segment 1015. Moreover, the user device 106 may identify one or more activity segemtns 1020 within the active segment 1015. In some cases, the user device 106 may receive past data over a longer period of time (e.g., hours) that includes one or more previous active segments 1015, such as one or more active segments 1015 and/or activity segments 1020 that occurred since data was last acquired from the ring (e.g., wearable device 104), such as data from earlier in the day. For example, the user device 106 may identify four separate activity segments 220 if the data was acquired at one time over the course of the entire time series (e.g., during inactive segment 1010-c).

The user device 106 may identify active segments 1015 and/or activity segments 1020 in a time series of data. In some implementations, the user device 106 may identify active segments 1015 and/or activity segments 1020 based on motion data and/or temperature data. In some implementations, the user device 106 may identify an activity segment 1020 based on an amount of motion and/or a duration of the motion. For example, the user device 106 may determine that the user is engaged in a physical activity if the data indicates that the user is involved in greater than a threshold amount of motion (e.g., acceleration or derived motion values). In some cases, the user device 106 may identify an activity segment 1020 when the intensity values, MET values, or regularity values are greater than a threshold value for a duration of time (e.g., a threshold amount of time).

The user device 106 may identify activity segments 1020 using temperature data (e.g., skin temperature). For example, the user device 106 may identify activity segments 1020 based on a change in temperature and/or a rate of change in temperature. In some cases, the user device 106 may identify activity segments 1020 based on a drop in user temperature during a period of time, such as a drop in user temperature that is greater than a threshold temperature drop. For example, the user device 106 may identify an activity segment 1020 when temperature drops by greater than a threshold amount within a defined period of time. The activity segment 1020 may be identified when the lower temperature is maintained for a threshold period of time. In some case, the temperature drops may be sustained during the periods of activity (e.g., during activity segments 1020). In such cases, the activity segments 1020 may include a drop in temperature from a starting temperature (e.g., a baseline temperature) down to a minimum temperature. The drop in temperature may be maintained during activity or may increase back towards the baseline. The temperature drops and increases may be due to external temperatures and/or the body's thermoregulatory response (e.g., blood flow and perspiration).

In some implementations, the user device 106 may identify activity segments 1020 using a combination of motion data and temperature data. For example, the user device 106 may identify an activity segment 1020 when the motion data and the temperature data for the segment satisfy a set of conditions (e.g., thresholds). An example set of conditions may include the presence of a threshold amount of motion (e.g., a threshold level of intensity) and a threshold temperature drop during a period of time. For example, as shown with reference to the activity segment 1020-a, the system 200 may identify the activity segment 1020-a based on an amount of motion being greater than or equal to some motion threshold for some time interval, and a corresponding drop in temperature during the time interval being greater than or equal to some temperature drop threshold.

In some cases, the user device 106 may identify activity segments 1020 based on motion data, temperature data, heart rate data, HRV data, and/or respiratory rate data. In some implementations, a user device 106, or other computing device, may acquire data used to identify activity segments 1020. For example, a user device 106 may acquire motion data (e.g., acceleration/gyro data) or other movement data (e.g., GPS data) that can be used to identify activity segments 1020.

In some cases, a rate of temperature change may vary based on a type of activity segment. In other words, different classified activity types may exhibit varying levels of temperature changes which a user may experience during an activity segment of the respective classified activity type. For example, a user may experience different temperature changes and/or different rates of temperature changes when biking as compared to temperature changes/rates of temperature changes when the user is running. As such, variance in temperature changes and rates of temperature change may be used to classify the type of activity (e.g., used to determine classified activity type).

For example, the server 110 (e.g., one or more classification modules) may receive features associated with temperature changes and change rates. The server 110 may identify different activities (e.g., different classified activity types) based on the received features. In some cases, the temperature changes and change rates may be affected based on whether the activity is outdoors/indoors (e.g., due to outdoor temperature), the level of intensity associated with the activity, the duration of the activity, or a combination thereof.

The user may perform an activity for a duration of time. The duration of time may be referred to as an "activity segment duration" or "segment duration." Each segment may be associated with one or more times that indicate when the activity/segment occurred. For example, each segment be associated with one or more segment time stamps that indicate the activity/segment start ("activity/segment start time"), activity/segment end ("activity/segment end time"), or other time (e.g., "activity/segment midpoint time"). In such cases, the activity segment 1020 may start at an activity start time, continue for an activity segment duration, and end at an activity end time. The data acquired during the segment duration may be referred to as "segment data" or "activity segment data." Example segment data may include motion data (e.g., accelerometer data, intensity values, etc.), temperature data, and other acquired data. In some implementations, the user device 106 may assign each activity segment 1020 a segment ID that the user device 106 and server 110 may use to identify the segment.

In some implementations, the computing devices may quickly identify the start of an activity and classify the activity due to the continuous monitoring of the user's physiological data (e.g., motion and temperature data). In some cases, the computing devices may acquire and analyze data over a longer time window (e.g., minutes or longer). Continuous monitoring and/or regular monitoring/analysis of user physiological data may result in segment data that includes data for a portion of an activity, such as when an activity is currently in progress. In other examples, segment data may include data for the entire duration of an activity, such as when segment data is collected for one or more previous activities. The system 200 may perform classification of segment data for segments that include partial data and/or complete data for an activity. In some cases, processing a smaller set of data (e.g., a short activity and/or partial data) may save processing resources and increase battery life. In some cases, processing larger sets of data (e.g., long activities and/or complete data) may increase the amount of processing, decrease battery life, but provide for greater classification accuracy. Although any of the processing described herein may be performed by a wearable device 104 (e.g., a ring), some of the processing may be performed by devices, such as mobile devices, personal computing devices, and/or servers.

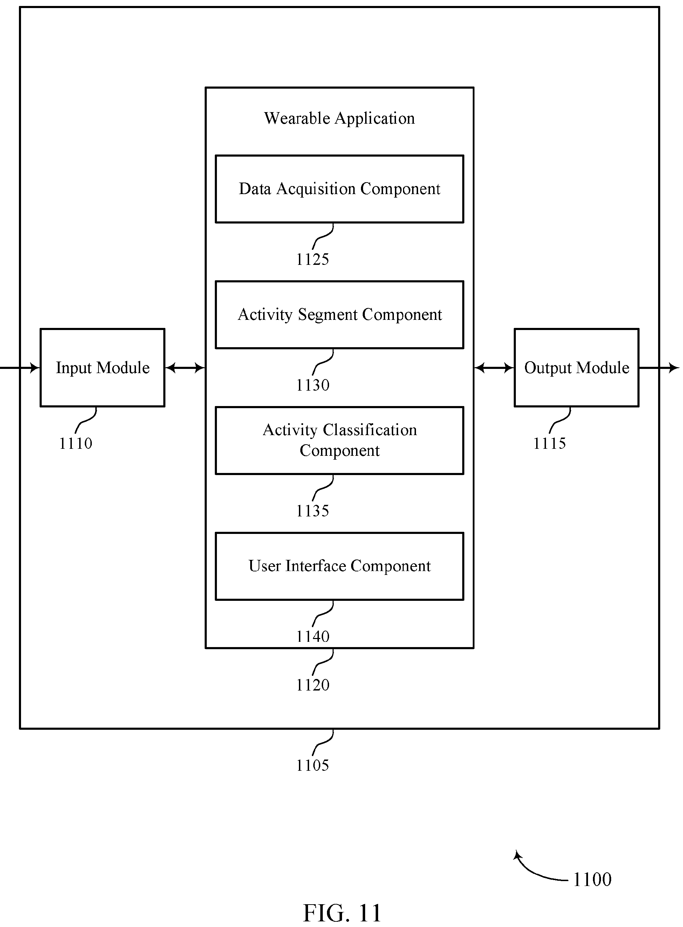

**FIG. 11** shows a block diagram 1100 of a device 1105 that supports activity classification and display in accordance with aspects of the present disclosure. The device 1105 may include an input module 1110, an output module 1115, and a wearable application 1120. The device 1105 may also include a processor. Each of these components may be in communication with one another (e.g., via one or more buses).

The input module 1110 may provide a means for receiving information such as packets, user data, control information, or any combination thereof associated with various information channels (e.g., control channels, data channels, information channels related to illness detection techniques). Information may be passed on to other components of the device 1105. The input module 1110 may utilize a single antenna or a set of multiple antennas.

The output module 1115 may provide a means for transmitting signals generated by other components of the device 1105. For example, the output module 1115 may transmit information such as packets, user data, control information, or any combination thereof associated with various information channels (e.g., control channels, data channels, information channels related to illness detection techniques). In some examples, the output module 1115 may be co-located with the input module 1110 in a transceiver module. The output module 1115 may utilize a single antenna or a set of multiple antennas.

For example, the wearable application 1120 may include a data acquisition component 1125, an activity segment component 1130, an activity classification component 1135, a user interface component 1140, or any combination thereof. In some examples, the wearable application 1120, or various components thereof, may be configured to perform various operations (e.g., receiving, monitoring, transmitting) using or otherwise in cooperation with the input module 1110, the output module 1115, or both. For example, the wearable application 1120 may receive information from the input module 1110, send information to the output module 1115, or be integrated in combination with the input module 1110, the output module 1115, or both to receive information, transmit information, or perform various other operations as described herein.

The wearable application 1120 may support classifying activity segments for a user in accordance with examples as disclosed herein. The data acquisition component 1125 may be configured as or otherwise support a means for receiving physiological data associated with the user via a wearable device, the physiological data comprising at least motion data. The activity segment component 1130 may be configured as or otherwise support a means for identifying, based at least in part on the motion data, an activity segment during which the user is engaged in a physical activity, wherein the activity segment is associated with activity segment data including at least the physiological data collected during the activity segment. The activity classification component 1135 may be configured as or otherwise support a means for generating activity classification data associated with the activity segment based at least in part on the activity segment data, the activity classification data including a plurality of classified activity types and corresponding confidence values, the confidence values indicating a confidence level associated with the corresponding classified activity type. The user interface component 1140 may be configured as or otherwise support a means for causing a GUI of a user device to display the activity segment data and at least one classified activity type of the plurality of classified activity types.

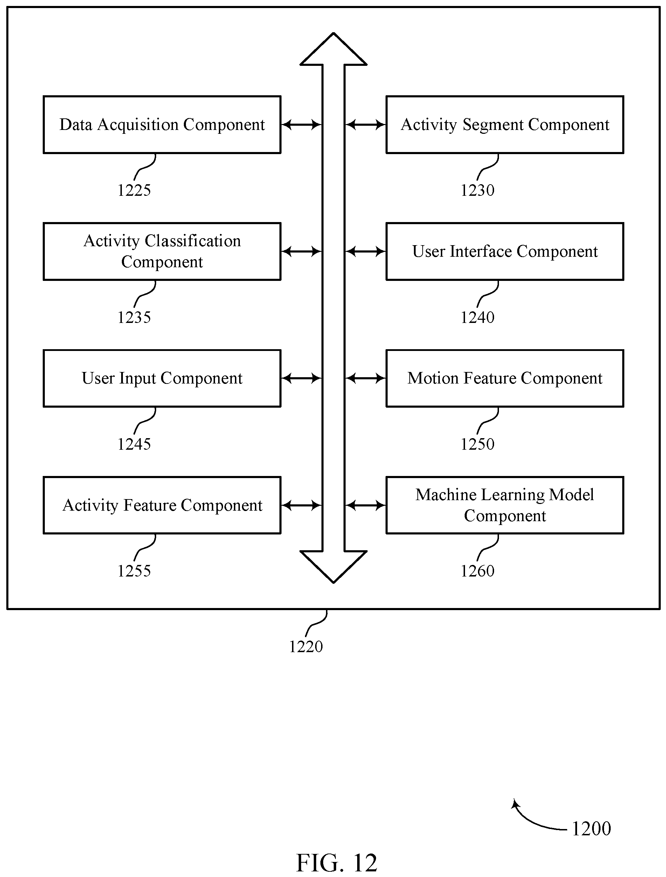

**FIG. 12** shows a block diagram 1200 of a wearable application 1220 that supports activity classification and display in accordance with aspects of the present disclosure. The wearable application 1220 may be an example of aspects of a wearable application or a wearable application 1120, or both, as described herein. The wearable application 1220, or various components thereof, may be an example of means for performing various aspects of activity classification and display as described herein. For example, the wearable application 1220 may include a data acquisition component 1225, an activity segment component 1230, an activity classification component 1235, a user interface component 1240, a user input component 1245, a motion feature component 1250, an activity feature component 1255, a machine learning model component 1260, or any combination thereof. Each of these components may communicate, directly or indirectly, with one another (e.g., via one or more buses).

The wearable application 1220 may support classifying activity segments for a user in accordance with examples as disclosed herein. The data acquisition component 1225 may be configured as or otherwise support a means for receiving physiological data associated with the user via a wearable device, the physiological data comprising at least motion data. The activity segment component 1230 may be configured as or otherwise support a means for identifying, based at least in part on the motion data, an activity segment during which the user is engaged in a physical activity, wherein the activity segment is associated with activity segment data including at least the physiological data collected during the activity segment. The activity classification component 1235 may be configured as or otherwise support a means for generating activity classification data associated with the activity segment based at least in part on the activity segment data, the activity classification data including a plurality of classified activity types and corresponding confidence values, the confidence values indicating a confidence level associated with the corresponding classified activity type. The user interface component 1240 may be configured as or otherwise support a means for causing a GUI of a user device to display the activity segment data and at least one classified activity type of the plurality of classified activity types.

In some examples, the user input component 1245 may be configured as or otherwise support a means for receiving, via the user device and in response to displaying the at least one classified activity type, a confirmation of the activity segment, wherein causing the GUI to display the activity segment data is based at least in part on receiving the confirmation.

In some examples, the confirmation comprises a confirmation of the at least one classified activity type, causing the GUI to display the activity segment data is based at least in part on receiving the confirmation of the at least one classified activity type.

In some examples, the user input component 1245 may be configured as or otherwise support a means for receiving, via the user device and in response to displaying the at least one classified activity type, one or more modifications for the activity segment, wherein causing the GUI to display the activity segment data is based at least in part on receiving the one or more modifications.

In some examples, the one or more modifications comprise an indication of an additional classified activity type associated with the activity segment.

In some examples, the activity segment component 1230 may be configured as or otherwise support a means for identifying the activity segment based at least in part on the temperature data.

In some examples, the activity segment component 1230 may be configured as or otherwise support a means for identifying the activity segment based at least in part on the motion data during the activity segment being greater than or equal to a motion threshold, and based at least in part on a temperature drop during the activity segment being greater than or equal to a threshold temperature drop.

In some examples, the motion feature component 1250 may be configured as or otherwise support a means for identifying one or more motion features based at least in part on the motion data. In some examples, the activity feature component 1255 may be configured as or otherwise support a means for identifying one or more temperature features based at least in part on the temperature data, wherein generating the activity classification data is based at least in part on the one or more motion features, the one or more temperature features, or both.

In some examples, the one or more motion features comprise an amount of motion during the activity segment. In some examples, the one or more temperature features comprise a temperature change during the activity segment, a rate of temperature change during the activity segment, or any combination thereof.

In some examples, the activity segment component 1230 may be configured as or otherwise support a means for identifying historical activity segment data for the user, the historical activity segment data comprising one or more historical activity segments for the user, wherein generating the activity classification data is based at least in part on the historical activity segment data.

In some examples, the confidence values associated with the plurality of classified activity types are based at least in part on the historical activity segment data.

In some examples, the machine learning model component 1260 may be configured as or otherwise support a means for inputting the activity segment data into a machine learning model, wherein generating the activity classification data is based at least in part on inputting the activity segment data into the machine learning model.

In some examples, the activity segment component 1230 may be configured as or otherwise support a means for identifying the activity segment based at least in part on one or more additional physiological parameters included within the physiological data, the one or more additional physiological parameters comprising heart rate data, HRV data, respiratory rate data, or any combination thereof.

In some examples, the wearable device comprises a wearable ring device.

In some examples, the wearable device collects the physiological data from the user based on arterial blood flow.

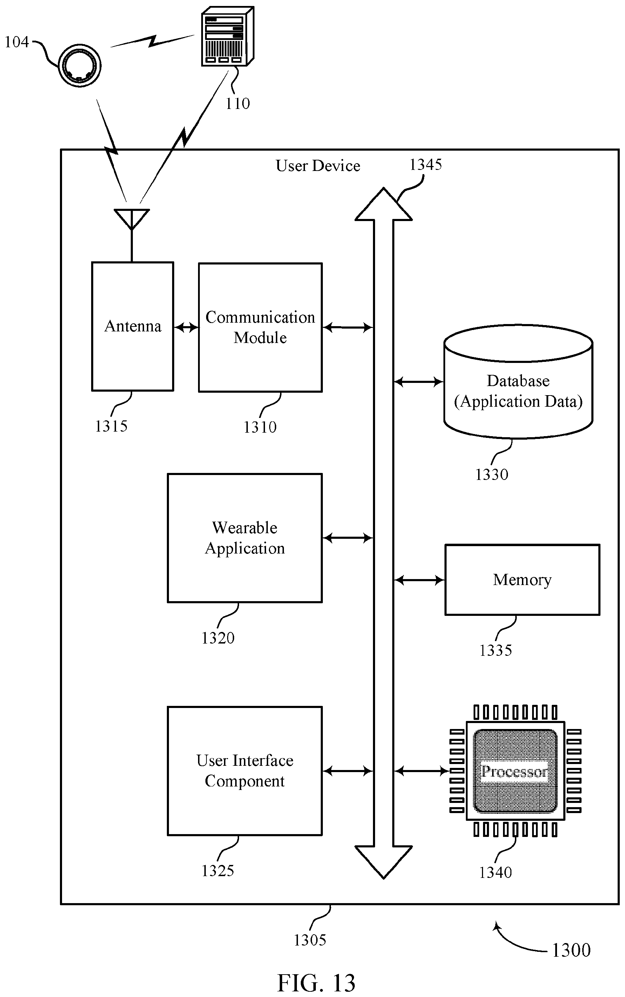

**FIG. 13** shows a diagram of a system 1300 including a device 1305 that supports activity classification and display in accordance with aspects of the present disclosure. The device 1305 may be an example of or include the components of a device 1105 as described herein. The device 1305 may include an example of a user device 106, as described previously herein. The device 1305 may include components for bi-directional communications including components for transmitting and receiving communications with a wearable device 104 and a server 110, such as a wearable application 1320, a communication module 1310, an antenna 1315, a user interface component 1325, a database (application data) 1330, a memory 1335, and a processor 1340. These components may be in electronic communication or otherwise coupled (e.g., operatively, communicatively, functionally, electronically, electrically) via one or more buses (e.g., a bus 1345).

The communication module 1310 may manage input and output signals for the device 1305 via the antenna 1315. The communication module 1310 may include an example of the communication module 220-b of the user device 106 shown and described in FIG. 2. In this regard, the communication module 1310 may manage communications with the ring 104 and the server 110, as illustrated in FIG. 2. The communication module 1310 may also manage peripherals not integrated into the device 1305. In some cases, the communication module 1310 may represent a physical connection or port to an external peripheral. In some cases, the communication module 1310 may utilize an operating system such as iOS^{®}, ANDROID^{®}, MS-DOS^{®}, MS-WINDOWS^{®}, OS/2^{®}, UNIX^{®}, LINUX^{®}, or another known operating system. In other cases, the communication module 1310 may represent or interact with a wearable device (e.g., ring 104), modem, a keyboard, a mouse, a touchscreen, or a similar device. In some cases, the communication module 1310 may be implemented as part of the processor 1340. In some examples, a user may interact with the device 1305 via the communication module 1310, user interface component 1325, or via hardware components controlled by the communication module 1310.

In some cases, the device 1305 may include a single antenna 1315. However, in some other cases, the device 1305 may have more than one antenna 1315, which may be capable of concurrently transmitting or receiving multiple wireless transmissions. The communication module 1310 may communicate bi-directionally, via the one or more antennas 1315, wired, or wireless links as described herein. For example, the communication module 1310 may represent a wireless transceiver and may communicate bi-directionally with another wireless transceiver. The communication module 1310 may also include a modem to modulate the packets, to provide the modulated packets to one or more antennas 1315 for transmission, and to demodulate packets received from the one or more antennas 1315.

The user interface component 1325 may manage data storage and processing in a database 1330. In some cases, a user may interact with the user interface component 1325. In other cases, the user interface component 1325 may operate automatically without user interaction. The database 1330 may be an example of a single database, a distributed database, multiple distributed databases, a data store, a data lake, or an emergency backup database.

The memory 1335 may include RAM and ROM. The memory 1335 may store computer-readable, computer-executable software including instructions that, when executed, cause the processor 1340 to perform various functions described herein. In some cases, the memory 1335 may contain, among other things, a BIOS which may control basic hardware or software operation such as the interaction with peripheral components or devices.

The processor 1340 may include an intelligent hardware device, (e.g., a general-purpose processor, a DSP, a CPU, a microcontroller, an ASIC, an FPGA, a programmable logic device, a discrete gate or transistor logic component, a discrete hardware component, or any combination thereof). In some cases, the processor 1340 may be configured to operate a memory array using a memory controller. In other cases, a memory controller may be integrated into the processor 1340. The processor 1340 may be configured to execute computer-readable instructions stored in a memory 1335 to perform various functions (e.g., functions or tasks supporting a method and system for sleep staging algorithms).

The wearable application 1320 may support classifying activity segments for a user in accordance with examples as disclosed herein. For example, the wearable application 1320 may be configured as or otherwise support a means for receiving physiological data associated with the user via a wearable device, the physiological data comprising at least motion data. The wearable application 1320 may be configured as or otherwise support a means for identifying, based at least in part on the motion data, an activity segment during which the user is engaged in a physical activity, wherein the activity segment is associated with activity segment data including at least the physiological data collected during the activity segment. The wearable application 1320 may be configured as or otherwise support a means for generating activity classification data associated with the activity segment based at least in part on the activity segment data, the activity classification data including a plurality of classified activity types and corresponding confidence values, the confidence values indicating a confidence level associated with the corresponding classified activity type. The wearable application 1320 may be configured as or otherwise support a means for causing a GUI of a user device to display the activity segment data and at least one classified activity type of the plurality of classified activity types.

By including or configuring the wearable application 1320 in accordance with examples as described herein, the device 1305 may support techniques for improved communication reliability, reduced latency, improved user experience related to reduced processing, reduced power consumption, more efficient utilization of communication resources, improved coordination between devices, longer battery life, and improved utilization of processing capability.

The wearable application 1320 may include an application (e.g., "app"), program, software, or other component which is configured to facilitate communications with a ring 104, server 110, other user devices 106, and the like. For example, the wearable application 1320 may include an application executable on a user device 106 which is configured to receive data (e.g., physiological data) from a ring 104, perform processing operations on the received data, transmit and receive data with the servers 110, and cause presentation of data to a user 102.

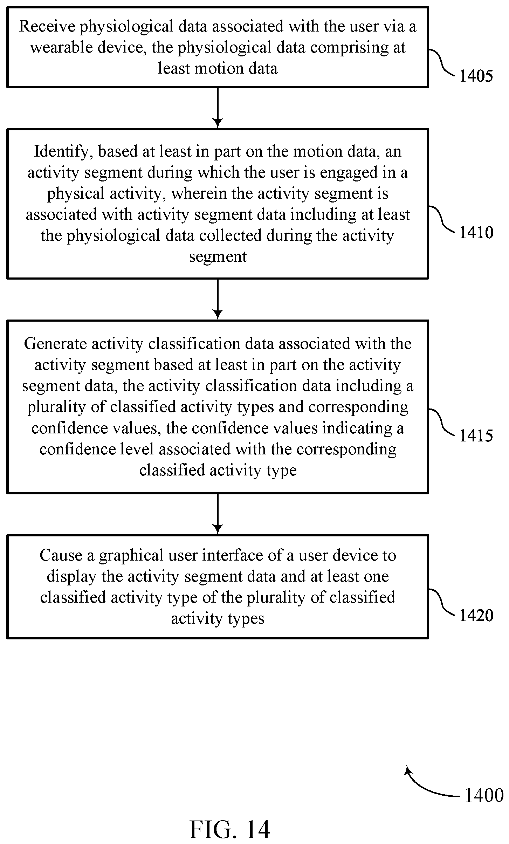

**FIG. 14** shows a flowchart illustrating a method 1400 that supports activity classification and display in accordance with aspects of the present disclosure. The operations of the method 1400 may be implemented by a user device or its components as described herein. For example, the operations of the method 1400 may be performed by a user device as described with reference to FIGs. 1 through 13. In some examples, a user device may execute a set of instructions to control the functional elements of the user device to perform the described functions. Additionally or alternatively, the user device may perform aspects of the described functions using special-purpose hardware.

At 1405, the method may include receiving physiological data associated with the user via a wearable device, the physiological data comprising at least motion data. The operations of 1405 may be performed in accordance with examples as disclosed herein. In some examples, aspects of the operations of 1405 may be performed by a data acquisition component 1225 as described with reference to FIG. 12.

At 1410, the method may include identifying, based at least in part on the motion data, an activity segment during which the user is engaged in a physical activity, wherein the activity segment is associated with activity segment data including at least the physiological data collected during the activity segment. The operations of 1410 may be performed in accordance with examples as disclosed herein. In some examples, aspects of the operations of 1410 may be performed by an activity segment component 1230 as described with reference to FIG. 12.

At 1415, the method may include generating activity classification data associated with the activity segment based at least in part on the activity segment data, the activity classification data including a plurality of classified activity types and corresponding confidence values, the confidence values indicating a confidence level associated with the corresponding classified activity type. The operations of 1415 may be performed in accordance with examples as disclosed herein. In some examples, aspects of the operations of 1415 may be performed by an activity classification component 1235 as described with reference to FIG. 12.

At 1420, the method may include causing a GUI of a user device to display the activity segment data and at least one classified activity type of the plurality of classified activity types. The operations of 1420 may be performed in accordance with examples as disclosed herein. In some examples, aspects of the operations of 1420 may be performed by a user interface component 1240 as described with reference to FIG. 12.

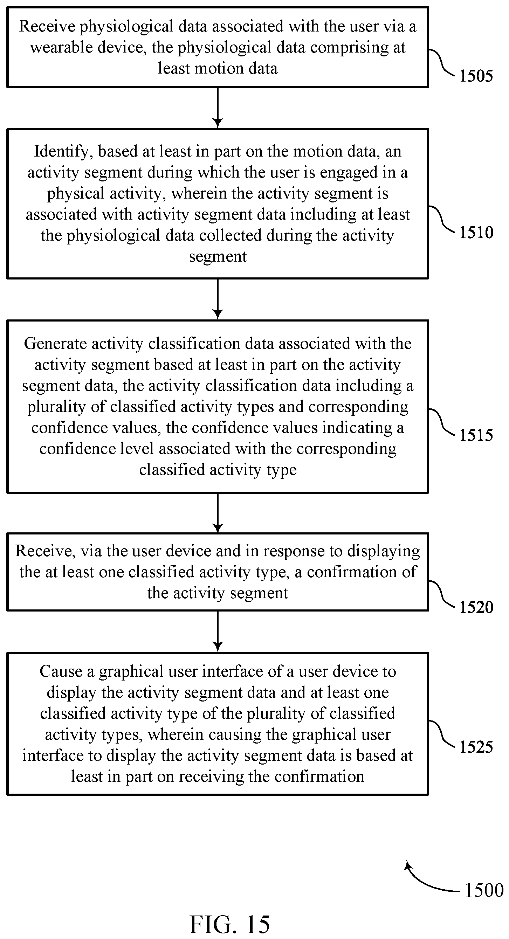

**FIG. 15** shows a flowchart illustrating a method 1500 that supports activity classification and display in accordance with aspects of the present disclosure. The operations of the method 1500 may be implemented by a user device or its components as described herein. For example, the operations of the method 1500 may be performed by a user device as described with reference to FIGs. 1 through 13. In some examples, a user device may execute a set of instructions to control the functional elements of the user device to perform the described functions. Additionally or alternatively, the user device may perform aspects of the described functions using special-purpose hardware.

At 1505, the method may include receiving physiological data associated with the user via a wearable device, the physiological data comprising at least motion data. The operations of 1505 may be performed in accordance with examples as disclosed herein. In some examples, aspects of the operations of 1505 may be performed by a data acquisition component 1225 as described with reference to FIG. 12.

At 1510, the method may include identifying, based at least in part on the motion data, an activity segment during which the user is engaged in a physical activity, wherein the activity segment is associated with activity segment data including at least the physiological data collected during the activity segment. The operations of 1510 may be performed in accordance with examples as disclosed herein. In some examples, aspects of the operations of 1510 may be performed by an activity segment component 1230 as described with reference to FIG. 12.

At 1515, the method may include generating activity classification data associated with the activity segment based at least in part on the activity segment data, the activity classification data including a plurality of classified activity types and corresponding confidence values, the confidence values indicating a confidence level associated with the corresponding classified activity type. The operations of 1515 may be performed in accordance with examples as disclosed herein. In some examples, aspects of the operations of 1515 may be performed by an activity classification component 1235 as described with reference to FIG. 12.

At 1520, the method may include receiving, via the user device and in response to displaying the at least one classified activity type, a confirmation of the activity segment. The operations of 1520 may be performed in accordance with examples as disclosed herein. In some examples, aspects of the operations of 1520 may be performed by a user input component 1245 as described with reference to FIG. 12.

At 1525, the method may include causing a GUI of a user device to display the activity segment data and at least one classified activity type of the plurality of classified activity types, wherein causing the GUI to display the activity segment data is based at least in part on receiving the confirmation. The operations of 1525 may be performed in accordance with examples as disclosed herein. In some examples, aspects of the operations of 1525 may be performed by a user interface component 1240 as described with reference to FIG. 12.

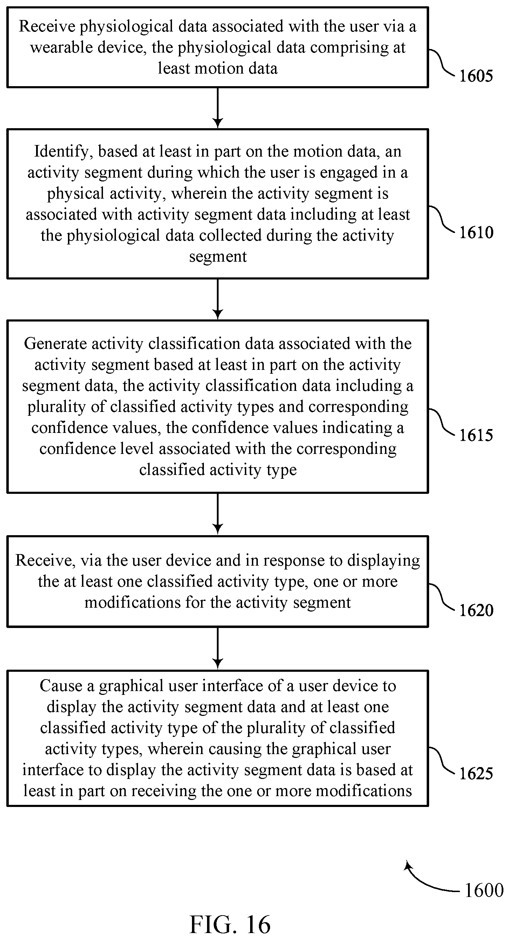

**FIG. 16** shows a flowchart illustrating a method 1600 that supports activity classification and display in accordance with aspects of the present disclosure. The operations of the method 1600 may be implemented by a user device or its components as described herein. For example, the operations of the method 1600 may be performed by a user device as described with reference to FIGs. 1 through 13. In some examples, a user device may execute a set of instructions to control the functional elements of the user device to perform the described functions. Additionally or alternatively, the user device may perform aspects of the described functions using special-purpose hardware.

At 1605, the method may include receiving physiological data associated with the user via a wearable device, the physiological data comprising at least motion data. The operations of 1605 may be performed in accordance with examples as disclosed herein. In some examples, aspects of the operations of 1605 may be performed by a data acquisition component 1225 as described with reference to FIG. 12.

At 1610, the method may include identifying, based at least in part on the motion data, an activity segment during which the user is engaged in a physical activity, wherein the activity segment is associated with activity segment data including at least the physiological data collected during the activity segment. The operations of 1610 may be performed in accordance with examples as disclosed herein. In some examples, aspects of the operations of 1610 may be performed by an activity segment component 1230 as described with reference to FIG. 12.

At 1615, the method may include generating activity classification data associated with the activity segment based at least in part on the activity segment data, the activity classification data including a plurality of classified activity types and corresponding confidence values, the confidence values indicating a confidence level associated with the corresponding classified activity type. The operations of 1615 may be performed in accordance with examples as disclosed herein. In some examples, aspects of the operations of 1615 may be performed by an activity classification component 1235 as described with reference to FIG. 12.

At 1620, the method may include receiving, via the user device and in response to displaying the at least one classified activity type, one or more modifications for the activity segment. The operations of 1620 may be performed in accordance with examples as disclosed herein. In some examples, aspects of the operations of 1620 may be performed by a user input component 1245 as described with reference to FIG. 12.

At 1625, the method may include causing a GUI of a user device to display the activity segment data and at least one classified activity type of the plurality of classified activity types, wherein causing the GUI to display the activity segment data is based at least in part on receiving the one or more modifications. The operations of 1625 may be performed in accordance with examples as disclosed herein. In some examples, aspects of the operations of 1625 may be performed by a user interface component 1240 as described with reference to FIG. 12.

It should be noted that the methods described above describe possible implementations, and that the operations and the steps may be rearranged or otherwise modified and that other implementations are possible. Furthermore, aspects from two or more of the methods may be combined.

A method for classifying activity segments for a user is described. The method may include receiving physiological data associated with the user via a wearable device, the physiological data comprising at least motion data, identifying, based at least in part on the motion data, an activity segment during which the user is engaged in a physical activity, wherein the activity segment is associated with activity segment data including at least the physiological data collected during the activity segment, generating activity classification data associated with the activity segment based at least in part on the activity segment data, the activity classification data including a plurality of classified activity types and corresponding confidence values, the confidence values indicating a confidence level associated with the corresponding classified activity type, and causing a GUI of a user device to display the activity segment data and at least one classified activity type of the plurality of classified activity types.

An apparatus for classifying activity segments for a user is described. The apparatus may include a processor, memory coupled with the processor, and instructions stored in the memory. The instructions may be executable by the processor to cause the apparatus to receive physiological data associated with the user via a wearable device, the physiological data comprising at least motion data, identify, based at least in part on the motion data, an activity segment during which the user is engaged in a physical activity, wherein the activity segment is associated with activity segment data including at least the physiological data collected during the activity segment, generate activity classification data associated with the activity segment based at least in part on the activity segment data, the activity classification data including a plurality of classified activity types and corresponding confidence values, the confidence values indicating a confidence level associated with the corresponding classified activity type, and cause a GUI of a user device to display the activity segment data and at least one classified activity type of the plurality of classified activity types.

Another apparatus for classifying activity segments for a user is described. The apparatus may include means for receiving physiological data associated with the user via a wearable device, the physiological data comprising at least motion data, means for identifying, based at least in part on the motion data, an activity segment during which the user is engaged in a physical activity, wherein the activity segment is associated with activity segment data including at least the physiological data collected during the activity segment, means for generating activity classification data associated with the activity segment based at least in part on the activity segment data, the activity classification data including a plurality of classified activity types and corresponding confidence values, the confidence values indicating a confidence level associated with the corresponding classified activity type, and means for causing a GUI of a user device to display the activity segment data and at least one classified activity type of the plurality of classified activity types.

A non-transitory computer-readable medium storing code for classifying activity segments for a user is described. The code may include instructions executable by a processor to receive physiological data associated with the user via a wearable device, the physiological data comprising at least motion data, identify, based at least in part on the motion data, an activity segment during which the user is engaged in a physical activity, wherein the activity segment is associated with activity segment data including at least the physiological data collected during the activity segment, generate activity classification data associated with the activity segment based at least in part on the activity segment data, the activity classification data including a plurality of classified activity types and corresponding confidence values, the confidence values indicating a confidence level associated with the corresponding classified activity type, and cause a GUI of a user device to display the activity segment data and at least one classified activity type of the plurality of classified activity types.

Some examples of the method, apparatuses, and non-transitory computer-readable medium described herein may further include operations, features, means, or instructions for receiving, via the user device and in response to displaying the at least one classified activity type, a confirmation of the activity segment, wherein causing the GUI to display the activity segment data may be based at least in part on receiving the confirmation.

In some examples of the method, apparatuses, and non-transitory computer-readable medium described herein, the confirmation comprises a confirmation of the at least one classified activity type, causing the GUI to display the activity segment data may be based at least in part on receiving the confirmation of the at least one classified activity type.

Some examples of the method, apparatuses, and non-transitory computer-readable medium described herein may further include operations, features, means, or instructions for receiving, via the user device and in response to displaying the at least one classified activity type, one or more modifications for the activity segment, wherein causing the GUI to display the activity segment data may be based at least in part on receiving the one or more modifications.

In some examples of the method, apparatuses, and non-transitory computer-readable medium described herein, the one or more modifications comprise an indication of an additional classified activity type associated with the activity segment.

Some examples of the method, apparatuses, and non-transitory computer-readable medium described herein may further include operations, features, means, or instructions for identifying the activity segment based at least in part on the temperature data.

Some examples of the method, apparatuses, and non-transitory computer-readable medium described herein may further include operations, features, means, or instructions for identifying the activity segment based at least in part on the motion data during the activity segment being greater than or equal to a motion threshold, and based at least in part on a temperature drop during the activity segment being greater than or equal to a threshold temperature drop.

In some examples of the method, apparatuses, and non-transitory computer-readable medium described herein, and the method, apparatuses, and non-transitory computer-readable medium may include further operations, features, means, or instructions for identifying one or more motion features based at least in part on the motion data and identifying one or more temperature features based at least in part on the temperature data, wherein generating the activity classification data may be based at least in part on the one or more motion features, the one or more temperature features, or both.

In some examples of the method, apparatuses, and non-transitory computer-readable medium described herein, the one or more motion features comprise an amount of motion during the activity segment and the one or more temperature features comprise a temperature change during the activity segment, a rate of temperature change during the activity segment, or any combination thereof.

Some examples of the method, apparatuses, and non-transitory computer-readable medium described herein may further include operations, features, means, or instructions for identifying historical activity segment data for the user, the historical activity segment data comprising one or more historical activity segments for the user, wherein generating the activity classification data may be based at least in part on the historical activity segment data.

In some examples of the method, apparatuses, and non-transitory computer-readable medium described herein, the confidence values associated with the plurality of classified activity types may be based at least in part on the historical activity segment data.

Some examples of the method, apparatuses, and non-transitory computer-readable medium described herein may further include operations, features, means, or instructions for inputting the activity segment data into a machine learning model, wherein generating the activity classification data may be based at least in part on inputting the activity segment data into the machine learning model.

Some examples of the method, apparatuses, and non-transitory computer-readable medium described herein may further include operations, features, means, or instructions for identifying the activity segment based at least in part on one or more additional physiological parameters included within the physiological data, the one or more additional physiological parameters comprising heart rate data, HRV data, respiratory rate data, or any combination thereof.

In some examples of the method, apparatuses, and non-transitory computer-readable medium described herein, the wearable device comprises a wearable ring device.

In some examples of the method, apparatuses, and non-transitory computer-readable medium described herein, the wearable device collects the physiological data from the user based on arterial blood flow.

The description set forth herein, in connection with the appended drawings, describes example configurations and does not represent all the examples that may be implemented or that are within the scope of the claims. The term "exemplary" used herein means "serving as an example, instance, or illustration," and not "preferred" or "advantageous over other examples." The detailed description includes specific details for the purpose of providing an understanding of the described techniques. These techniques, however, may be practiced without these specific details. In some instances, well-known structures and devices are shown in block diagram form in order to avoid obscuring the concepts of the described examples.

In the appended figures, similar components or features may have the same reference label. Further, various components of the same type may be distinguished by following the reference label by a dash and a second label that distinguishes among the similar components. If just the first reference label is used in the specification, the description is applicable to any one of the similar components having the same first reference label irrespective of the second reference label.

Information and signals described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

The various illustrative blocks and modules described in connection with the disclosure herein may be implemented or performed with a general-purpose processor, a DSP, an ASIC, an FPGA or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices (e.g., a combination of a DSP and a microprocessor, multiple microprocessors, one or more microprocessors in conjunction with a DSP core, or any other such configuration).

The functions described herein may be implemented in hardware, software executed by a processor, firmware, or any combination thereof. If implemented in software executed by a processor, the functions may be stored on or transmitted over as one or more instructions or code on a computer-readable medium. Other examples and implementations are within the scope of the disclosure and appended claims. For example, due to the nature of software, functions described above can be implemented using software executed by a processor, hardware, firmware, hardwiring, or combinations of any of these. Features implementing functions may also be physically located at various positions, including being distributed such that portions of functions are implemented at different physical locations. Also, as used herein, including in the claims, "or" as used in a list of items (for example, a list of items prefaced by a phrase such as "at least one of" or "one or more of") indicates an inclusive list such that, for example, a list of at least one of A, B, or C means A or B or C or AB or AC or BC or ABC (i.e., A and B and C). Also, as used herein, the phrase "based on" shall not be construed as a reference to a closed set of conditions. For example, an exemplary step that is described as "based on condition A" may be based on both a condition A and a condition B without departing from the scope of the present disclosure. In other words, as used herein, the phrase "based on" shall be construed in the same manner as the phrase "based at least in part on."

Computer-readable media includes both non-transitory computer storage media and communication media including any medium that facilitates transfer of a computer program from one place to another. A non-transitory storage medium may be any available medium that can be accessed by a general purpose or special purpose computer. By way of example, and not limitation, non-transitory computer-readable media can comprise RAM, ROM, electrically erasable programmable ROM (EEPROM), compact disk (CD) ROM or other optical disk storage, magnetic disk storage or other magnetic storage devices, or any other non-transitory medium that can be used to carry or store desired program code means in the form of instructions or data structures and that can be accessed by a general-purpose or special-purpose computer, or a general-purpose or special-purpose processor. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, include CD, laser disc, optical disc, digital versatile disc (DVD), floppy disk and Blu-ray disc where disks usually reproduce data magnetically, while discs reproduce data optically with lasers. Combinations of the above are also included within the scope of computer-readable media.

The description herein is provided to enable a person skilled in the art to make or use the disclosure. Various modifications to the disclosure will be readily apparent to those skilled in the art, and the generic principles defined herein may be applied to other variations without departing from the scope of the disclosure. Thus, the disclosure is not limited to the examples and designs described herein, but is to be accorded the broadest scope consistent with the principles and novel features disclosed herein. The claims of the parent application are reproduced below as clauses. These clauses define preferable combinations of features. The applicant reserves the right to pursue protection for these combinations of features, and/or any other subject-matter contained in the parent application as filed, either in the present divisional application or in a further application divided from the present divisional application. The claims of the parent application are not the claims of the current application which are contained in a separate section headed "claims".
1. A method for classifying activity segments for a user, comprising:
   receiving physiological data associated with the user via a wearable device, the physiological data comprising at least motion data;
   identifying, based at least in part on the motion data, an activity segment during which the user is engaged in a physical activity, wherein the activity segment is associated with activity segment data including at least the physiological data collected during the activity segment;
   generating activity classification data associated with the activity segment based at least in part on the activity segment data, the activity classification data including a plurality of classified activity types and corresponding confidence values, the confidence values indicating a confidence level associated with the corresponding classified activity type; and
   causing a graphical user interface of a user device to display the activity segment data and at least one classified activity type of the plurality of classified activity types.
2. The method of claim 1, further comprising:
   receiving, via the user device and in response to displaying the at least one classified activity type, a confirmation of the activity segment, wherein causing the graphical user interface to display the activity segment data is based at least in part on receiving the confirmation.
3. The method of claim 2, wherein the confirmation comprises a confirmation of the at least one classified activity type, and wherein causing the graphical user interface to display the activity segment data is based at least in part on receiving the confirmation of the at least one classified activity type.
4. The method of claim 1, further comprising:
   receiving, via the user device and in response to displaying the at least one classified activity type, one or more modifications for the activity segment, wherein causing the graphical user interface to display the activity segment data is based at least in part on receiving the one or more modifications.
5. The method of claim 4, wherein the one or more modifications comprise an indication of an additional classified activity type associated with the activity segment.
6. The method of claim 1, wherein the physiological data further includes temperature data, the method further comprising:
   identifying the activity segment based at least in part on the temperature data.
7. The method of claim 6, further comprising:
   identifying the activity segment based at least in part on the motion data during the activity segment being greater than or equal to a motion threshold, and based at least in part on a temperature drop during the activity segment being greater than or equal to a threshold temperature drop.
8. The method of claim 1, wherein the physiological data further comprises temperature data, the method further comprising:
   identifying one or more motion features based at least in part on the motion data; and
   identifying one or more temperature features based at least in part on the temperature data, wherein generating the activity classification data is based at least in part on the one or more motion features, the one or more temperature features, or both.
9. The method of claim 8, wherein the one or more motion features comprise an amount of motion during the activity segment, and wherein the one or more temperature features comprise a temperature change during the activity segment, a rate of temperature change during the activity segment, or any combination thereof.
10. The method of claim 1, further comprising:
   identifying historical activity segment data for the user, the historical activity segment data comprising one or more historical activity segments for the user, wherein generating the activity classification data is based at least in part on the historical activity segment data.
11. The method of claim 10, wherein the confidence values associated with the plurality of classified activity types are based at least in part on the historical activity segment data.
12. The method of claim 1, further comprising:
   inputting the activity segment data into a machine learning model, wherein generating the activity classification data is based at least in part on inputting the activity segment data into the machine learning model.
13. The method of claim 1, further comprising:
   identifying the activity segment based at least in part on one or more additional physiological parameters included within the physiological data, the one or more additional physiological parameters comprising heart rate data, heart rate variability data, respiratory rate data, or any combination thereof.
14. The method of claim 1, wherein the wearable device comprises a wearable ring device.
15. The method of claim 1, wherein the wearable device collects the physiological data from the user based on arterial blood flow.
16. An apparatus for classifying activity segments for a user, comprising:
   a processor;
   memory coupled with the processor; and
   instructions stored in the memory and executable by the processor to cause the apparatus to:
      receive physiological data associated with the user via a wearable device, the physiological data comprising at least motion data;
      identify, based at least in part on the motion data, an activity segment during which the user is engaged in a physical activity, wherein the activity segment is associated with activity segment data including at least the physiological data collected during the activity segment;
      generate activity classification data associated with the activity segment based at least in part on the activity segment data, the activity classification data including a plurality of classified activity types and corresponding confidence values, the confidence values indicating a confidence level associated with the corresponding classified activity type; and
      cause a graphical user interface of a user device to display the activity segment data and at least one classified activity type of the plurality of classified activity types.
17. The apparatus of claim 16, wherein the instructions are further executable by the processor to cause the apparatus to:
   receive, via the user device and in response to displaying the at least one classified activity type, a confirmation of the activity segment, wherein causing the graphical user interface to display the activity segment data is based at least in part on receiving the confirmation.
18. The apparatus of claim 17, wherein the confirmation comprises a confirmation of the at least one classified activity type, and wherein causing the graphical user interface to display the activity segment data is based at least in part on receiving the confirmation of the at least one classified activity type.
19. The apparatus of claim 16, wherein the instructions are further executable by the processor to cause the apparatus to:
   receive, via the user device and in response to displaying the at least one classified activity type, one or more modifications for the activity segment, wherein causing the graphical user interface to display the activity segment data is based at least in part on receiving the one or more modifications.
20. A non-transitory computer-readable medium storing code for classifying activity segments for a user, the code comprising instructions executable by a processor to:
   receive physiological data associated with the user via a wearable device, the physiological data comprising at least motion data;
   identify, based at least in part on the motion data, an activity segment during which the user is engaged in a physical activity, wherein the activity segment is associated with activity segment data including at least the physiological data collected during the activity segment;
   generate activity classification data associated with the activity segment based at least in part on the activity segment data, the activity classification data including a plurality of classified activity types and corresponding confidence values, the confidence values indicating a confidence level associated with the corresponding classified activity type; and
   cause a graphical user interface of a user device to display the activity segment data and at least one classified activity type of the plurality of classified activity types.

## Claims

1. A method for classifying activity segments for a user, comprising:
receiving physiological data associated with the user via a wearable device, the physiological data comprising at least motion data;
identifying, based at least in part on the motion data, an activity segment during which the user is engaged in a physical activity, wherein the activity segment is associated with activity segment data including at least the physiological data collected during the activity segment;
generating activity classification data associated with the activity segment based at least in part on the activity segment data, the activity classification data including a plurality of classified activity types and corresponding confidence values, the confidence values indicating a confidence level associated with the corresponding classified activity type; and
receiving from the user, via the user device (106), one or more user-input modifications for the activity segment, wherein the one or more user-input modifications include a modification of the classified activity type, a time of activity, an intensity of activity, or any combination thereof;
causing a graphical user interface of a user device to display the activity segment data and at least one classified activity type of the plurality of classified activity types, wherein causing the graphical user interface to display the activity segment data is based at least in part on receiving the one or more user-input modifications.

2. The method of claim 1, further comprising:
generating one or more scores for the user based at least in part on the activity segment data, the at least one classified activity type, or both; and
modifying the one or more scores for the user based at least in part on the one or more user-input modifications for the activity segment.

3. The method of claim 1, further comprising:
receiving, via the user device and in response to displaying the at least one classified activity type, a confirmation of the activity segment, wherein causing the graphical user interface to display the activity segment data is based at least in part on receiving the confirmation.

4. The method of claim 3, wherein the confirmation comprises a confirmation of the at least one classified activity type, and wherein causing the graphical user interface to display the activity segment data is based at least in part on receiving the confirmation of the at least one classified activity type.

5. The method of claim 1, wherein the physiological data further includes temperature data, the method further comprising:
identifying the activity segment based at least in part on the temperature data.

6. The method of claim 5, further comprising:
identifying the activity segment based at least in part on the motion data during the activity segment being greater than or equal to a motion threshold, and based at least in part on a temperature drop during the activity segment being greater than or equal to a threshold temperature drop.

7. The method of claim 1, wherein the physiological data further comprises temperature data, the method further comprising:
identifying one or more motion features based at least in part on the motion data; and
identifying one or more temperature features based at least in part on the temperature data, wherein generating the activity classification data is based at least in part on the one or more motion features, the one or more temperature features, or both.

8. The method of claim 7, wherein the one or more motion features comprise an amount of motion during the activity segment, and wherein the one or more temperature features comprise a temperature change during the activity segment, a rate of temperature change during the activity segment, or any combination thereof.

9. The method of claim 1, further comprising:
inputting the activity segment data into a machine learning model, wherein generating the activity classification data is based at least in part on inputting the activity segment data into the machine learning model.

10. The method of claim 9, further comprising:
training the machine learning model based at least in part on the activity segment data, the at least one classified activity type, and the one or more user-input modifications for the activity segment.

11. The method of claim 1, further comprising:
identifying the activity segment based at least in part on one or more additional physiological parameters included within the physiological data, the one or more additional physiological parameters comprising heart rate data, heart rate variability data, respiratory rate data, or any combination thereof.

12. An apparatus for classifying activity segments for a user, comprising:
a processor;
memory coupled with the processor; and
instructions stored in the memory and executable by the processor to cause the apparatus to:
receive physiological data associated with the user via a wearable device, the physiological data comprising at least motion data;
identify, based at least in part on the motion data, an activity segment during which the user is engaged in a physical activity, wherein the activity segment is associated with activity segment data including at least the physiological data collected during the activity segment;
generate activity classification data associated with the activity segment based at least in part on the activity segment data, the activity classification data including a plurality of classified activity types and corresponding confidence values, the confidence values indicating a confidence level associated with the corresponding classified activity type; and
receive from the user, via the user device (106), one or more user-input modifications for the activity segment, wherein the one or more user-input modifications include a modification of the classified activity type, a time of activity, an intensity of activity, or any combination thereof;
cause a graphical user interface of a user device to display the activity segment data and at least one classified activity type of the plurality of classified activity types, wherein causing the graphical user interface to display the activity segment data is based at least in part on receiving the one or more user-input modifications.

13. The apparatus of claim 12, wherein the instructions are further executable by the processor to cause the apparatus to:
generate one or more scores for the user based at least in part on the activity segment data, the at least one classified activity type, or both; and
modify the one or more scores for the user based at least in part on the one or more user-input modifications for the activity segment.

14. The apparatus of claim 12, wherein the instructions are further executable by the processor to cause the apparatus to:
input the activity segment data into a machine learning model, wherein generating the activity classification data is based at least in part on inputting the activity segment data into the machine learning model.

15. The apparatus of claim 14, wherein the instructions are further executable by the processor to cause the apparatus to:
train the machine learning model based at least in part on the activity segment data, the at least one classified activity type, and the one or more user-input modifications for the activity segment.
